# EUROPÄISCHE PATENTSCHRIFT

(11) **EP 2 167 685 B1**
(45) Veröffentlichungstag und Bekanntmachung des Hinweises auf die Patenterteilung: **17.12.2014**
(21) Anmeldenummer: 08761031.7
(22) Anmeldetag: 13.06.2008
(51) Int. Cl.: C12Q 1/68

(54) **VERFAHREN UND SONDEN/PRIMERSYSTEM ZUM "REAL TIME" NACHWEIS EINES NUKLEINSÄURETARGETS**
METHOD AND PROBE/PRIMER SYSTEM FOR THE "REAL TIME" DETECTION OF A NUCLEIC ACID TARGET
PROCÉDÉ ET SYSTÈME DE SONDES ET D'AMORCES POUR LA MISE EN ÉVIDENCE EN TEMPS RÉEL D'UN D'ACIDE NUCLÉIQUE CIBLE

(30) Priorität: 13.06.2007 DE 102007027779; 29.06.2007 DE 102007031137; 08.02.2008 DE 102008008485
(43) Veröffentlichungstag der Anmeldung: 31.03.2010
(73) Patentinhaber: Attomol GmbH Molekulare Diagnostika, 03205 Bronkow (DE)
(72) Erfinder: LEHMANN, Werner, 03205 Bronkow (DE); HANSCHMANN, Henning, 03205 Calau (DE); SYRING, Mandy, 03205 Lipten (DE)
(74) Vertreter: Gulde & Partner
(86) Internationale Anmeldenummer: PCT/EP2008/057512
(87) Internationale Veröffentlichungsnummer: WO 2008/152144

(56) Entgegenhaltungen:
- EP-A- 0 566 751
- WO-A-03/072810
- WO-A-03/102239
- WO-A-2004/061127
- WO-A-2005/047468
- GB-A- 2 338 301
- US-A1- 2007 020 656
- MONIS ET AL: "Nucleic acid amplification-based techniques for pathogen detection and identification" INFECTION, GENETICS AND EVOLUTION, ELSEVIER, AMSTERDAM, NL, Bd. 6, Nr. 1, 1. Januar 2006 (2006-01-01), Seiten 2-12, XP005221546 ISSN: 1567-1348

## Beschreibung

Die Erfindung betrifft allgemein das Gebiet der Nukleinsäureamplifikation. Insbesondere betrifft sie ein Verfahren zum "real time"-Nachweis eines Nukleinsäuretargets. Gegenstand der Erfindung sind auch ein Primer-/Sondensystem und ein Kit zum Nachweis eines Nukleinsäuretargets und seine Verwendung in einer PCR.

Verfahren zur Erkennung und Mengenbestimmung von Nukleinsäuren sind bekannt. Ausgehend von einer zu analysierenden mRNA wird zunächst eine einzelsträngige cDNA mit Hilfe einer Reversen Transkriptase hergestellt, mit Hilfe einer PCR wird dann ein doppelsträngiges DNA-Amplifikationsprodukt erzeugt. Die PCR ist sowohl für qualitative als auch für quantitative Aussagen einsetzbar. Eine Mengenbestimmung von PCR-Produkten kann auf unterschiedliche Art erfolgen. Neuere Detektionsmethoden erlauben es, die in-vitro-Synthese von PCR-Produkten "real time", d.h. homogen direkt im jeweils verwendeten PCR-Reaktionsgefäß zu messen. Diese so genannte "real time"-PCR stellt eine besonders sensitive Methode dar. Hierbei wird in jedem Zyklus der PCR die Entstehung von PCR-Produkten verfolgt. Die Messung der Amplifikation erfolgt dabei in der Regel in Thermozyklern, welche zusätzliche Mittel zur Messung von Fluoreszenzsignalen während der Amplifikationsreaktion aufweisen. Die Amplifikationsprodukte werden beispielsweise durch Fluoreszenz-markierte Hybridisierungssonden, die lediglich bei Bindung an die Targetnukleinsäure Fluoreszenzsignale emittieren oder in bestimmten Fällen auch durch Doppelstrang-DNA bindende Fluoreszenzfarbstoffe detektiert. Ein definierter Signalschwellenwert wird für alle analysierten Reaktionen festgelegt und die zur Erreichung des Schwellenwerts notwendige Zykluszahl Cp sowohl für die Targetnukleinsäure als auch für die Referenznukleinsäuren bestimmt. Auf der Grundlage der für die Targetnukleinsäure sowie die Referenznukleinsäure erhaltenen Cp-Werte können so entweder absolute oder relative Kopienzahlen des Targetmoleküls bestimmt werden.

Ein neueres Verfahren zum Nachweis eines spezifischen Nukleinsäuremoleküls verwendet so genannte "Molecular Beacons" (Tyagi et al. US 6,150,097A). Molecular Beacons sind farbstoffmarkierte Oligonukleotide, die eine Stamm-Schleifen-Struktur haben. An den beiden freien Enden der Stamm-Abschnitte (dem 3'- und dem 5'-Ende) ist jeweils ein Fluorophor gekoppelt, wobei der eine als Reporter-Farbstoff und der andere als Quencher-Farbstoff, der die Fluoreszenz des Reporter-Farbstoffs bei hinreichender räumlicher Nähe durch Foerster-Energietransfer löscht. Die Sequenzen der Stammabschnitte an beiden Enden der Molecular Beacons sind so gewählt, dass dann, wenn sich der Molecular Beacon faltet, die Stammabschnitte ausschließlich aneinander, nicht aber mit anderen Abschnitten des Oligonukleotids hybridisieren. Der Abstand zwischen Reporter- und Quencher-Farbstoff ist im Zustand der hybridisierten Stammabschnitte hinreichend klein, so dass der Fluoreszenz-Farbstoff auch bei geeigneter Anregung mit Licht nicht fluoresziert. Der Schleifen-Abschnitt weist eine Sequenz auf, die zur Sequenz einer Targetsequenz komplementär ist. Befinden sich die Molecular Beacons und die Targetsequenz aufweisenden Nukleinsäure-Moleküle in einer Lösung, können die Schleifenabschnitte und die Targetsequenz-Abschnitte hybridisieren, wodurch sich der Molecular Beacon unter Lösung der Hybridisierung der beiden Stammabschnitte entfaltet. Diese Entfaltung führt zur Vergrößerung des räumlichen Abstands zwischen dem Reporter-Farbstoff und dem Quencher-Farbstoff und der Reporter-Farbstoff wird zur Fluoreszenz angeregt. Bei kontinuierlicher Beobachtung der Fluoreszenz-Intensität kann ein Anstieg der Intensität festgestellt werden, wenn die Molecular Beacons die Targetsequenzen aufspüren und an diesen hybridisieren. So können die Nukleinsäuremoleküle quantitativ nachgewiesen werden. Diese Molecular Beacons haben den großen Nachteil einer aufwändigen und teuren Synthese, da diese Sonden sowohl mit dem Fluoreszenz- als auch mit dem Quencher-Farbstoff markiert werden müssen. Quencher-Fluoreszenzfarbstoff-Systeme werden in vergleichbaren Sondensystemen wie Amplifluor-Primer, Scorpions und TaqMan-Sonden genutzt.

EP 0566751 beschreibt ein Verfahren zum Nachweis einer Nukleinsäure, welches darauf beruht, daß ein fluoreszenzmarkierter Primer nach Polymerasevermittelter, sequenzspezifischer Verlängerung eine intramolekulare Haarnadelstruktur bildet und eine targespezifische, fluoreszenzmashicke Sonde so an die verlängerte Sequenz bindet, daß die Fluoreszenzmaskierungen in enge Nachbarschaft kommen und eine Fluoreszenztransfer entsteht, der gemessen wird.

Ein weiteres bekanntes System ist das Light-Cycler-System. Hier werden die Fluoreszenzfarbstoffe auf zwei verschiedene Oligonukleotide verteilt. Am 3'-Ende des ersten Oligonukleotids befindet sich der eine Fluoreszenzfarbstoff, z.B. Fluorescein, und das sich stromabwärts anlagernde Oligonukleotid hat am 5'-Ende einen weiteren Fluoreszenzfarbstoff, z.B. LC Red 640. Fluorescein wird von einer LED als Lichtquelle angeregt und emittiert Licht, das über Fluoreszenzresonanzenergietransfer (FRET) das zweite Fluoreszenzmolekül anregt. Das von dem zweiten Farbstoff emittierte Licht wird über einen entsprechenden Filter detektiert. Eine Anregung des zweiten Farbstoffs kann nur dann erfolgen, wenn sich durch die Anlagerung der beiden Oligonukleotide an ihren Komplementärstrang die beiden Farbstoffmoleküle in räumlicher Nähe (Distanz 1 bis 5 Nukleotide) befinden. Das Prinzip dieses Systems beruht auf der sekundären Anregung eines zweiten Fluoreszenzfarbstoffs, die erst durch die Generation des PCR-Produktes ermöglicht wird.

Die genannten Systeme sind grundsätzlich für Multiplexanwendungen einsetzbar, haben jedoch den Nachteil, dass - sollen verschiedene Zielsequenzen nebeneinander nachgewiesen werden - der Reaktionsansatz wegen der steigenden Zahl der verschiedenen Detektionssonden relativ teuer wird und die zunehmende Zahl der Sonden die Amplifikationsreaktion beeinträchtigen kann. Außerdem sind nicht genügend FRET-Paare bekannt, um einen hohen Multiplex-Grad zu erzielen. Die bisher existierenden Sondensysteme erlauben es auch nicht größere Sequenzabschnitte der Targetsequenz zu analysieren, ohne dass mehrere fluoreszenzmarkierte, und damit kostenintensive, Sonden synthetisiert werden müssen. Die Detektion mehrerer Targetsequenzen umfaßt auch die gleichzeitige Detektion von einer Targetsequenz und einem internen Standard oder mehrerer interner Standards in einer PCR, wobei die mehreren Standards für die Kalibration der Quantifizierung der Targetsequenz auch in unterschiedlichen Konzentrationen vorliegen können.

Der Erfindung lag deshalb die Aufgabe zugrunde, Verfahren und Primer-/SondenSysteme bereitzustellen, die kostengünstiger und einfach synthetisiert werden können und die gleiche oder verbesserte Sensitivität aufweisen. Durch geeignete Veränderung der Primerstrukturen soll eine Möglichkeit gefunden werden, den teuren Einsatz jeweils spezifischer Sondenoligonukleotide zu umgehen, wobei die Multiplex-Anwendung, die für die heutige Diagnostik immer wichtiger wird, nicht beeinträchtigt werden soll auch wenn größere Sequenzbereiche analysiert werden.

Erfindungsgemäß wird die Aufgabe gelöst durch ein Verfahren zum "real-time"-Nachweis von Nukleinsäuretargets als Produkte einer Nukleinsäure-Amplifikationsreaktion mittels eines neuen Primer-/Sondensystems. Dieses Verfahren wird in Anspruch 1 definiert.

Dabei gelangen Fluorophore 1 und 2 in eine für den FRET ausreichende Nähe, so dass sie bei Anregung durch Licht entsprechender Wellenlänge eine Detektion der Fluoreszenz des Akzeptors ermöglichen. Das heißt, einer der beiden Fluorophore wird durch Licht geeigneter Wellenlänge angeregt, so dass zwischen Fluorophor 1 und 2 ein FRET stattfindet, der fluoreszenzoptisch detektiert wird. Eine solche ausreichende Nähe zwischen den beiden Fluorophoren liegt z.B. dann vor, wenn die beiden Fluorophore 1 und 2 nicht mehr als 10 Nukleotide voneinander getrennt vorliegen, bevorzugt nicht mehr als 5 Nukleotide, ganz besonders bevorzugt beträgt der Abstand zwischen Fluorophor 1 und 2 ein bis 5 Nukleotide. Dabei spielt es keine Rolle, ob Fluorophor 2 stromabwärts oder stromaufwärts von Fluorophor 1 im entsprechenden Abstand zum liegen kommt. Überraschenderweise wird der FRET zwischen Fluorophor 1 und 2 auch nicht dadurch beeinträchtigt, dass erfindungsgemäß Fluorophor 1 und Fluorophor 2 auf gegenläufigen Strängen positioniert sind.

Mittel und Wege zur Anregung der Fluorophore mit Licht geeigneter Wellenlänge und Geräte und Verfahren zur Detektion der Fluoreszenz nach erfolgtem FRET sind dem Fachmann bekannt.

Ebenso sind dem Fachmann Techniken, Mittel und Wege bekannt erfindungsgemäße Primer und Sonden herzustellen und gegebenenfalls mit Fluorophor1 oder 2 zu versehen. Markierungsreaktionen zur kovalenten Kopplung von Primern oder Sonden mit entsprechenden Fluorophoren sind dem Fachmannn ebenso bekannt, wie geeignete Bedingungen zur Hybridisierung von z.B. mit Fluorophor 1 markierten Sonden mit erfindungsgemäßen Primern.

Erfindungsgemäße Primer zeichnen sich dadurch aus, dass diese eine enzymatische Verlängerung an ihrem 3'-Ende erlauben, gegebenenfalls können die erfindungsgemäßen Primer eine oder mehrere Modifikationen an ihrem 3'-Ende tragen, welche eine enzymatische Verlängerung des 3'-Endes komplementär zum Target erlauben. Solche Modifikationen sind dem Fachmann bekannt. Besonders bevorzugt besteht eine solche Modifikation in einer freien Hydroxygruppe der terminalen Base am 3'-Ende des erfindungsgemäßen Primers, welche sich zur enzymatischen Verlängerung eignet.

Geeignete Mittel und Wege zur enzymatischen Verlängerung der erfindungsgemäßen Primer an ihrem 3'-Ende komplementär zum Target sind dem Fachmann bekannt. Eine solche enzymatische Verlängerung kann z.B. durch Verwendung einer geeigneten DNA-Polymerase vermittelt werden. Entsprechende Reaktionsbedingungen sind dem Fachmann bekannnt.

Eine Target-spezifische Sequenz (TSS) ist eine Nukleotidsequenz, die zu einem bestimmten Abschnitt der Targetsequenz komplementär ist und eine spezifische Hybridisierung zwischen dem Targetstrang und TSS-tragendem Primer- oder Sondenstrang ermöglicht. Bevorzugt hat eine TSS eine Länge von 3 bis 100 Nukleotiden, besonders bevorzugt von 3 bis 50, ganz besonders bevorzugt von 5 bis 35 Nukleotiden.

In einer besonderen Ausführungsform sind die zwei verschiedenen, jeweils mit einem FRET-Donor bzw. FRET-Akzeptor markierten Primer-/Sonden-Oligonukleotide so konstruiert, dass sich nach erfolgter Hybridisierung durch Ausbildung einer intramolekularen Haarnadel zwischen einer Haarnadelbildungssequenz (HBS) des mindestens einen Primers und des zur HBS komplementären Abschnitts des enzymatisch verlängerten mindestens einen Primers, die Fluorophore 1 und 2 in einer für den FRET ausreichenden Nähe befinden, so dass sie während eines PCR-Zyklus eine Fluoreszenz des Akzeptors ermöglichen. Das heißt, einer der beiden Fluorophore wird durch Licht geeigneter Wellenlänge angeregt, so dass zwischen Fluorophor 1 und 2 ein FRET stattfindet, der fluoreszenzoptisch detektiert wird. Eine solche ausreichende Nähe zwischen den beiden Fluorophoren liegt z.B. dann vor, wenn die beiden Fluorophore 1 und 2 nicht mehr als 10 Nukleotide voneinander getrennt vorliegen, bevorzugt nicht mehr als 5 Nukleotide, ganz besonders bevorzugt zwischen 1 bis 5 Nukleotide. Wird die erforderliche Nähe zwischen Fluorophor 1 und Fluorophor 2 über eine Haarnadelstruktur vermittelt, ist die räumliche Nähe der beiden Fluorophore unabhängig von dem tatsächlichen Nukleotidabstand auf der linearen Sequenz des Stranges, der mit Sonde 2 hybridisert. Dabei spielt es keine Rolle, ob Fluorophor 2 stromabwärts oder stromaufwärts von Fluorophor 1 im entsprechenden Abstand zum liegen kommt. Überraschenderweise wird der FRET zwischen Fluorophor 1 und 2 auch nicht dadurch beeinträchtigt, dass erfindungsgemäß Fluorophor 1 und Fluorophor 2 auf gegenläufigen Strängen positioniert sind.

Eine Haarnadelbildungssequenz (HBS) ist eine Nukleotidsequenz, die eine Sequenz aufweist, die zu einer stomabwärts oder stromaufwärts gelegenen weiteren Nukleotidsequenz desselben Strangmoleküls komplementär ist und eine intramolekulare Hybridisierung der beiden komplementären Strangbereiche erlaubt. So wird eine intramolekulare sog. Haarnadelstruktur gebildet. Bevorzugt besitzt die HBS eine Sequenz die im Wesentlichen identisch ist mit einem Sequenzabschnitt des Targets, wobei dieser Targetabschnitt (auch Komplement zu TBS 2 genannt) stromabwärts von der targetspezifischen Bindungsstelle des Primers (dem Komplement zu TSS 1) in Richtung der enzymatischen Verlängerung des HBS-tragenden Primers oder Sonde liegt. Bevorzugt hat eine HBS eine Länge von 3 bis 100 Nukleotiden, besonders bevorzugt von 3 bis 50, ganz besonders bevorzugt von 3 bis 35 Nukleotiden, bzw. von 3 bis 20 Nukleotiden.

Ein solches Verfahren ist im folgenden beschrieben:
Verfahren zum Echtzeit-Nachweis von Nukleinsäuretargets als Produkte einer Nukleinsäure-Amplifikationsreaktion mittels einem Primer/Sondensystem umfassend die Schritte:
   a) Einsatz von mindestens einem Primer, der
      - mindestens eine für die zu amplifizierende Nukleinsäure targetspezifische Sequenz (TSS),
      - sowie eine Haarnadelbildungssequenz (HBS) umfasst,
      - der am 3'-Ende eine enzymatische Verlängerung komplementär zum Target gestatten und
      - die Einführung eines Fluorophores 1 durch kovalente Bindung oder durch Bindung einer Sonde 1 erlaubt.
   b) Einführung mindestens eines Fluorophor 1 durch kovalente Bindung an den mindestens einen Primer aus Schritt a) oder durch Hybridisierung einer Sonde 1, die mit Fluorophor 1 markiert ist, mit dem mindestens einen Primer aus Schritt a).
   c) Enzymatische Verlängerung des Primers am 3'-Ende.
   d) Einsatz von mindestens einer Sonde 2, die mindestens am 3'-Ende ein Fluorophor 2 trägt, wobei die eine Sonde mit dem am 3'-Ende verlängerten Primer hybridisiert, sodass Fluophor 1 und Fluophor 2 so in räumliche Nähe gelangen, dass ein Fluoreszenzresonanzenergietransfer zwischen den Fluophoren 1 und 2 möglich ist.
   e) Ausbildung von mindestens einer intramolekularen oder intermolekularen Haarnadel durch Hybridisierung der HBS des gemäß Schritt c) verlängerten mindestens einem Primer mit dem zur HBS komplementären Abschnitt des verlängerten Primer oder mit einem zur HBS komplementären Abschnitt von Sonde 2.
   f) Anregung eines der beiden Fluorophore durch Licht geeigneter Wellenlänge, so dass zwischen den Fluorophoren 1 und 2 ein Fluoreszenzresonanzenergietransfer stattfindet.
   g) Fluoreszenzoptische Detektion der Fluoreszenz.

In einer besonderen Ausführungsvariante der Erfindung weist der Loop der gebildeten Haarnadelstruktur eine Sequenzlänge von mindestens 3 Nukleotiden auf, bevorzugt von mehr als 5 Nukleotiden, besonders bevorzugt von mehr als 10 Nukleotiden, weiter bevorzugt von mehr als 20 Nukleotiden, ganz besonders bevorzugt von 25 oder mehr Nukleotiden, oder wobei die Sequenzlänge der Loop-Struktur ausgewählt ist aus einem Bereich von 25 bis 60 Nukleotiden.

In dem erfindungsgemäßen Verfahren können noch eine oder mehrere weitere Sonden zum Einsatz kommen, die markiert oder unmarkiert sein könnnen und die zumindest teilweise innerhalb der Loop-Sequenz der gebildeten Haarnadelstruktur an den Strang des verlängerten mindestens einen Primers spezifisch binden könnnen. Solche weiteren Sonden können vor, während oder nach dem Einsatz der mit Fluorophor 2 markierten Sonde 2 mit dem verlängerten mindestens einen Primer hybridisiert werden. Dabei ist die Sequenz der mindestens einen weiteren Sonde so gewählt, dass bei erfolgter Hybridisierung dieser weiteren Sonde an den verlängerten Primer, die Haarnadelstruktur so verändert wird, dass zwischen Fluorophor 1 und Fluorophor 2 kein FRET mehr stattfinden kann. Dies ist z.B. dann der Fall, wenn die Haarnadelstruktur durch die Bindung der mindestens einen weiteren Sonde destabilisiert wird oder die Harrnadelstruktur sogar aufgelöst wird. Die Destabilisierung der Haarnadel bzw. die Auflösung der Haarnadel kann dadurch erreicht werden, dass die spezifische Bindungssequenz der mindestens einen weiteren Sonde mindestens teilweise mit der Stammregion der Haarnadel überlappt. Die weitere Sonde kann auch mit der in die Haarnadel hineinreichenden Sequenz von Primer 1 überlappen.

Daneben kann an die Haarnadel eine bevorzugt am 3'-Ende blockierte oder am 3'-Ende nicht komplementäre Sonde (Blockadesonde), die bevorzugt keinen Fluoreszenzfarbstoff trägt, hybridisieren und zur Auflösung der Haarnadel und Aufhebung des für einen FRET erforderlichen Abstandes zwischen Fluorophor 1 und 2 beitragen. Alternativ oder in Kombination zu dieser Sonde können Teilsequenzen ko-amplifizierter Targetsequenzen wie z.B. Pathogensequenzen oder interne Standards in der Haarnadel hybridisieren.

Durch differentielle Hybridisierung z.B. während der Aufzeichnung von Schmelzkurven ist es möglich, Sequenzpolymorphismen unter Verwendung einer mindestens einen weiteren Sonde, bevorzugt mindestens einer nicht markierten Blockadesonde oder ko-amplifizierter Targetsequenzen über weite Sequenzbereiche der Haarnadel zu identifizieren.

In einer weiteren Ausführungsvariante der Erfindung weist der mindestens eine Primer am 5'-Ende zusätzlich einen zum Target unspezifischen Schwanz auf. Fluorophor 1 kann zum Beispiel am spezifischen oder unspezifischen Teil eines ersten Primers oder an einem weiteren Primer vorliegen. Die Anordnung der Fluorophore ist dabei austauschbar, so dass Fluorophor 1 an einem Primer und Fluorophor 2 an der Sonde vorliegen kann bzw. umgekehrt. Alternativ können Fluorophor 1 und 2 in Sonden eingebracht werden. In welcher Richtung FRET erfolgt bzw. detektiert wird hat keinen Einfluss auf das erfindungsgemäße Verfahren, beide Richtungen sind von der Erfindung umfasst. Vorzugsweise liegen die Fluorophore 1 und 2 kovalent gebunden an Primern und Sonden vor.

Die Erfindung basiert auf einem neuartigen Primer-/Sonden-Design, wobei in einer ganz besonders bevorzugten Variante des mindestens einen Primer dieser eine Teilsequenz aufweist, die im 5'-Bereich teilweise komplementär zu seinem 3'-Bereich ist, so dass sie eine intramolekulare Haarnadel bildet.

Vorzugsweise kann der mindestens eine Primer das Fluorophor 1 am 5'-Ende aufweisen, wobei der Primer entweder nur aus einer Target-spezifischen Sequenz besteht oder zusätzlich einen unspezifischen Schwanz besitzt. Nach enzymatischer Verlängerung und erfolgter Sondenhybridisierung bildet zum Nachweis des Targets der Primer am 5'-Ende partiell bzw. vollständig eine Haarnadel mit sich selbst oder zu der komplementär zum Target verlängerten Primersequenz, so dass ein FRET-Signal entsteht.

In einer weiteren Ausführungsform der Erfindung ist das 5'-Ende des Primers Targetunspezifisch und komplementär zum 3'-Ende einer Sonde, die ihrerseits mit ihrem 5'-Bereich an das Target hybridisiert. Dabei bildet sich eine haarnadelartige Loop-Struktur zwischen verlängertem Primer und Sonde aus. Fluorophor 1 am 5'-Ende Primers und Fluorophor 2 bevorzugt am 3'-Ende der Sonde, die somit gleichzeig blockiert ist, kommen durch die Loop-Bildung in räumliche Nähe, so dass FRET stattfindet.
Alternativ kann das sequenzunspezifsche, mit Fluophor 1 markierte 5'-Ende des Primers auch an das komplentäre, nicht Target-spezifische Ende der 5'-Ende der Sonde hybridisieren. Auch in diesem Falle tritt FRET ein, wenn die Sonde im Bereich des 5'-Endes Fluophor 2 trägt. Natürlich ist es auch möglich, dass der Primer im Bereich des 5'-Endes mit Fluophor 1 markiert ist und die Sonde am 5'-Ende mit Fluophor 2. Fluophor 2 kann auch am Primer gebunden sein, wenn die Sonde Fluophor 2 trägt.
Es ist ebenfalls vorgesehen, dass Primer 1 ein Sondenprimer ist, der aus einem Targetsequenz-spezifischen 3'- und einem Targetsequenz-unspezifischen 5'-Abschnitt besteht und in der PCR spezifitätsbestimmend ist. Ein Primer 2 der eines der beiden Fluophore trägt und sequenzgleich mit dem Targetsequenz-unspezifischen Abschnitt des Sondenprimers ist, bestimmt dagegen die Sensitivität der Amplifikation. Dadurch ist es möglich, im Multiplex-Ansatz unter Verwendung verschiedener Sondenprimer und eines universellen Primer 2 der in alle Amplikons kostengünstig ein Fluorophor einfügt. Auf diese Weise können auch alle anderen Ausführungsvarianten für den Multiplex-Einsatz erweitert werden.

In einer ganz bevorzugten erfindungsgemäßen Ausgestaltung ist das Verfahren dadurch gekennzeichnet, dass es mindestens drei Primer verwendet. Dabei umfasst es die folgenden Schritte:
In einer ersten Reaktion erfolgt der Einsatz eines Primerpaares 1 und 2. Der gewählte Vorwärtsprimer 1 umfasst mindestens eine Targetbindungssequenz 1 (TSS 1) und mindestens eine Haarnadelbildungssequenz (HBS) und ist so konstruiert, dass TSS 1 an das zu amplifizierende Target binden kann. Anschließend wird dieser Primer 1 komplementär zum Target verlängert, Rückwärtsprimer 2 bindet an das komplementäre Target und wird ebenfalls verlängert. Primer 1 enthält vorzugsweise eine TSS 1 und eine HBS. In einer Variante enthält er mehrere HBS, die sich geringfügig bis vollständig unterscheiden. So können in einer Reaktion mehrere Targetsequenzen in Summe erfasst werden (z.B. Erfassung humaner Papillomaviren der Gruppen "hohes Risiko" und "geringes Risiko").
In einer weiteren Reaktion wird ein Primer 3 eingesetzt, welcher mit dem 5'-Bereich von Primer 1 identisch oder teilweise identisch ist, jedoch keine TSS aufweist. Primer 3 trägt *gegebenenfalls* das Fluorophor 1 am 5'-Ende . Er ist so konstruiert, dass er eine Haarnadel 1 vollständig oder teilweise mit sich selbst oder mit komplementären Abschnitten der Primer 1-Sequenz bilden kann. Die Bedingungen werden so eingestellt, dass mit Primer 2 und 3 eine enzymatische Verlängerung und ggf. eine Amplifikation in einem oder mehreren Zyklen stattfindet. Dabei bindet die mindestens eine HBS von Primer 1 an einen oder mehrere weitere(n) Targetbindungsabschnitt(e), welche(r) nun zwischen verlängertem Primer 1 und der komplementären Sequenz von Primer 2 liegt, wodurch eine Haarnadel 2 entsteht. Wird das Amplifikat dann mit der mit Fluorophor 2 markierten Sonde in Kontakt gebracht, kann das mit dem Fluorophor 1 gebildete FRET-Signal detektiert werden. In einer bevorzugten Ausgestaltung trägt der Primer 3 Fluorophor 1 und die Sonde mit Fluorophor 2 am 3'-Ende hybrisidisiert vollständig an der Targetsequenz. Alternativ kann die Sonde mit Fluorophor 2 am 3'-Ende über Haarnadel 2 und Primer 1 an der Targetssequenz hybridisieren. Die Loop-Sequenz in Haarnadel 2 beträgt bevorzugt 10-100 Nuklotide kann aber in Abhängigkeit der Analysefragestellung auch 50-1000 Nukleotide betragen.

Ein solches erfindungsgemäßes Vefahren ist im folgenden dargestellt:
a) in einer ersten Reaktion,
   - Einsatz eines Primerpaares 1 und 2, wobei
   - der Vorwärtsprimer 1 mindestens eine Targetbindungssequenz 1 (TSS 1) und mindestens eine Haarnadelbildungssequenz (HBS) umfasst, wobei die HBS identisch ist zu einem Abschnitt der Targetsequenz, der sich zwischen der TSS 1 und der Bindungsstelle des Rückwärtsprimer 2 befindet,
   - die TSS 1 des Vorwärtsprimer 1 an das zu amplifizierende Target bindet,
   - anschließend Vorwärtsprimer 1 komplementär zum Target enzymatisch verlängert wird, und
   - danach Rückwärtsprimer 2 an das Produkt der Verlängerung des Vorwärtsprimers 1 bindet und ebenfalls enzymatisch verlängert wird.
b) in einer weiteren Reaktion,
   - Einsatz eines Primer 3, welcher
      - mit dem 5'-Bereich von Primer 1 identisch oder teilweise identisch ist,
      - das Fluorophor 1 trägt, und
      - wobei Primer 3 so konstruiert ist, dass er eine erste intramolekulare Haarnadel vollständig oder teilweise mit sich selbst oder mit entsprechenden Abschnitten der Primer 1-Sequenz bilden kann, und
   - Enzymatische Verlängerung des Primer 3 unter Verwendung des verlängerten Rückwärtsprimers 2 aus Schritt a) als Matritze.
c) Einsatz von mindestens einer Sonde 2, die mindestens am 3'-Ende ein Fluorophor 2 trägt, wobei die Sonde 2 mit dem verlängerten Primer 3 hybridisiert, sodass Fluophor 1 und Fluophor 2 so in räumliche Nähe gelangen, dass ein Fluoreszenzresonanzenergietransfer zwischen den Fluophoren möglich ist.
d) Ausbildung einer zweiten intramolekularen Haarnadel im verlängerten Primer 3 aus Schritt b) durch Hybridisierung der mindestens einen HBS der ursprünglichen Primer 1-Sequenz mit dem zu dieser HBS komplementären Abschnitt des verlängerten Primer 3.
e) Anregung eines der beiden Fluorophore durch Licht geeigneter Wellenlänge, so dass zwischen den Fluorophoren 1 und 2 ein Fluoreszenzresonanzenergietransfer stattfindet.
f) Fluoreszenzoptische Detektion der Fluoreszenz.

In einer besonderen Ausführungsvariante der Erfindung weist der Loop der gebildeten Haarnadelstruktur 2 eine Sequenzlänge von mindestens 3 Nukleotiden auf, bevorzugt von mehr als 5 Nukleotiden, besonders bevorzugt von mehr als 10 Nukleotiden, weiter bevorzugt von mehr als 20 Nukleotiden, ganz besonders bevorzugt von 25 oder mehr Nukleotiden, oder wobei die Sequenzlänge der Loop-Struktur ausgewählt ist aus einem Bereich von 25 bis 60 Nukleotiden.

In dem erfindungsgemäßen Verfahren können noch eine oder mehrere weitere Sonden zum Einsatz kommen, die markiert oder unmarkiert sein könnnen und die zumindest teilweise innerhalb der Loop-Sequenz der gebildeten Haarnadelstruktur 2 an den Strang des verlängerten Primers 3 spezifisch binden könnnen. Solche weiteren Sonden können vor, während oder nach dem Einsatz der mit Fluorophor 2 markierten Sonde 2 mit dem verlängerten Primer 3 hybridisiert werden. Dabei ist die Sequenz der mindestens einen weiteren Sonde so gewählt, dass bei erfolgter Hybridisierung dieser weiteren Sonde an den verlängerten Primer 3, die Harrnadelstruktur so verändert wird, dass zwischen Fluorophor 1 und Fluorophor 2 kein FRET mehr stattfinden kann. Dies ist z.B. dann der Fall, wenn die Haarnadelstruktur durch die Bindung der mindestens einen weiteren Sonde destabilisiert wird oder die Harrnadelstruktur sogar aufgelöst wird. Die Destabilisierung der Haarnadel bzw. die Auflösung der Haarnadel kann dadurch erreicht werden, dass die spezifische Bbindungssequenz der mindestens einen weiteren Sonde mindestens teilweise mit der Stammregion der Haarnadel überlappt.
Daneben kann in Haarnadel 1 oder 2 eine bevorzugt am 3'-Ende blockierte oder am 3'-Ende nicht komplementäre Sonde (Blockadesonde), die bevorzugt keinen Fluoreszenzfarbstoff trägt, hybridisieren und zur Auflösung der Haarnadel und Aufhebung des für einen FRET erforderlichen Abstandes zwischen Fluorophor 1 und 2 beitragen. Alternativ oder in Kombination zu dieser Sonde können Teilsequenzen ko-amplifizierter Targetsequenzen wie z.B. Pathogensequenzen oder interne Standards in Haarnadel 1 oder 2 hybridisieren.

Durch differentielle Hybridisierung z.B. während der Aufzeichnung von Schmelzkurven ist es möglich, Sequenzpolymorphismen unter Verwendung einer mindestens einen weiteren Sonde, bevorzugt mindestens einer nicht markierten Blockadesonde oder ko-amplifizierter Targetsequenzen über weite Sequenzbereiche der Haarnadel zu identifizieren.

In einer weiteren Ausgestaltung der Erfindung können die drei Primer und zwei Sonden verwendet werden, die jeweils FRET-Donor und -Akzeptor (Fluorophor 1 und 2) tragen, wobei die eingesetzte Sonde 1 Primer-1-spezifisch ist und die verwendete Sonde 2 Target-spezifisch.

Die erfindungsgemäß eingesetzten Fluorophore 1 und 2 sind ausgewählt aus Fluoreszenzfarbstoffmolekülen, wobei das Licht, das vom ersten Fluorophor absorbiert und remittiert wird, vom zweiten, nach Bindung der Sonde(n) dem ersten Fluorophor räumlich nahen zweiten Fluorophor erneut absorbiert und remittiert wird. Vorzugsweise dienen verschiedene Fluorophore als Donor und Akzeptor, so dass ein FRET-Signal als Auftreten Donor-stimulierter Fluoreszenz des Akzeptors aufgezeichnet werden kann. Alternativ kann auch derselbe Farbstoff als Donor und Akzeptor eingesetzt werden und die resultierende Depolarisation der Fluoreszenz detektiert werden. Die Farbstoffe können auch so ausgewählt werden, daß bei Kontakt ein strahlungsloser Energietransfer stattfindet. Bevorzugt ist Fluorophor 1 Fluorescein und Fluorophor 2 LC Red 640 oder LC Red 705 Beispiele für weitere bevorzugte FRET-Paare sind:

| | |
|---|---|
| Fluorophor A | FRET-Partner B |
| Aminocumarin | Fluorescein |
| Fluorescein | Rhodamin oder Tetramethylrhodamin |
| Fluorescein | Cy3 |
| Cy3 | Cy5 |
| Cy3 | Tetramethylrhodamin |
| Europium | Cy5 |
| Terbium | Rhodamin |
| Bodipy | Bodipy |
| Dansyl | Fluorescein |
| Naphtalen | Dansyl |
| Ruthenium | Cy5. |

Wie bereits ausgeführt, kann erfindungsgemäß Fluorophor 1 an einem Primer und Fluorophor 2 an der Sonde vorliegen bzw. umgekehrt. Das FRET-Paar kann auch an Sonden gebunden vorliegen. Die erfindungsgemäß eingesetzen FRET-Paare erzielen einen hohen Multiplex-Grad. Es sind für das erfindungsgemäße Verfahren zahlreiche Fluorophore für den Spektralbereich von 350 bis 750 nm, das heißt von Ultraviolett bis Infrarot, verfügbar.

Die Amplifikationsreaktion ist vorzugsweise eine homogene Flüssigphasen-PCR, eine Multiplex-PCR, eine asymmetrische PCR, eine Festphasen-PCR, eine RT-PCR, eine in situ-PCR, eine immuno-PCR oder eine nested PCR.

Das Verfahren zum Echtzeit-Nachweis von Nukleinsäuretargets als Produkt einer Nukleinsäure-Amplifikationsreaktion mittels der neuartigen Primer-/Sondensysteme zum Nachweis von Nukleinsäuren umfasst (siehe Beispiel 1):
a) Einsatz von mindestens einem Primer, der mindestens eine für die zu amplifizierende Nukleinsäure targetspezifische Sequenz (TSS) sowie eine Haarnadelbildungssequenz (HBS) umfasst und der am 3'-Ende eine enzymatische Verlängerung komplementär zum Target gestattet und die Einführung eines Fluorophores 1 durch kovalente Bindung oder durch Bindung einer Sonde 1 vermittelt,
b) Enzymatische Verlängerung des Primers am 3'-Ende,
c) Ausbildung von mindestens einer intramolekularen Haarnadel durch Hybridisierung der HBS mit dem verlängerten Primer, wodurch Fluorophor 1 in räumliche Nähe zur Bindungsstelle von Sonde 2 gebracht wird,
d) Einsatz von mindestens einer Sonde 2, die mindestens am 3'-Ende ein Fluorophor 2 trägt, wobei die mindestens eine Sonde 2 mit dem am 3'-Ende verlängerten Primer hybridisiert,
e) Anregung eines der beiden Fluorophore durch Licht geeigneter Wellenlänge, so dass zwischen den Fluorophoren 1 und 2 ein Fluoreszenzresonanzenergietransfer stattfindet
f) Fluoreszenzoptische Detektion der Fluoreszenz.

Vorzugsweise kommen für die verschiedenen Amplikons oder Polymorphismen unterschiedliche FRET-Paare zum Einsatz.

Ein bevorzugtes Verfahren mittels der erfindungsgemäßen Primer-/Sondensysteme umfasst:
a) Einsatz von mindestens einem Primer, der mindestens eine für die zu amplifizierende Nukleinsäure targetspezifische Sequenz (TSS) sowie eine Haarnadelbildungssequenz (HBS) umfasst und der am 3'-Ende eine enzymatische Verlängerung komplementär zum Target gestattet und die Einführung eines Fluorophores 1 durch kovalente Bindung oder durch Bindung einer Sonde 1 vermittelt,
b) Enzymatische Verlängerung des Primers am 3'-Ende,
c) Einsatz von mindestens einer Sonde 2, die mindestens am 3'-Ende Targetsequenz-unspezifisch ist und ein Fluorophor 2 trägt, wobei die mindestens eine Sonde 2 mit dem am 3'-Ende verlängerten Primer hybridisiert,
d) Ausbildung von mindestens einer intramolekularen Haarnadel durch Hybridisierung der HBS mit dem 3'-Ende der am verlängerten Primer hybridisierten Sonde 2, wodurch Fluorophor 1 in räumliche Nähe zum Fluorophor 2 von Sonde 2 gebracht wird,
e) Anregung eines der beiden Fluorophore durch Licht geeigneter Wellenlänge, so dass zwischen den Fluorophoren 1 und 2 ein Fluoreszenzresonanzenergietransfer stattfindet
f) Fluoreszenzoptische Detektion der Fluoreszenz.

Ein weiteres bevorzugtes Verfahren mittels der erfindungsgemäßen Primer-/Sondensysteme umfasst:
a) Einsatz von mindestens einem Primer, der mindestens eine für die zu amplifizierende Nukleinsäure targetspezifische Sequenz (TSS) sowie eine Haarnadelbildungssequenz (HBS) umfasst und der am 3'-Ende eine enzymatische Verlängerung komplementär zum Target gestattet und die Einführung eines Fluorophores 1 durch kovalente Bindung oder durch Bindung einer Sonde 1 vermittelt,
b) Enzymatische Verlängerung des Primers am 3'-Ende,
c) Ausbildung von mindestens einer intramolekularen Haarnadel durch Hybridisierung der HBS mit dem verlängerten Primer, wodurch Fluorophor 1 in räumliche Nähe zur Bindungsstelle von Sonde 2 gebracht wird,
d) Bindung einer Blockadesonde in Abhängigkeit der Sequenz der Haarnadel
e) Einsatz von mindestens einer Sonde 2, die mindestens am 3'-Ende ein Fluorophor 2 trägt, wobei die mindestens eine Sonde mit dem am 3'-Ende verlängerten Primer hybridisiert,
f) Anregung eines der beiden Fluorophore durch Licht geeigneter Wellenlänge, so dass zwischen den Fluorophoren 1 und 2 ein Fluoreszenzresonanzenergietransfer stattfindet
g) Fluoreszenzoptische Detektion der Fluoreszenz.

Ist die Schmelztemperatur der Blockadesonde von der Haarnadelsequenz geringer als die Schmeltztemperatur der Haarnadel selbst, führt die Bindung der Blockadesonde zur Öffnung der Haarnadel bereits unterhalb der Schmelztemperatur der Haarnadel. Die Hybridisierungseigenschaften und somit die Eigenschaften der Haarnadelsequenz, insbesondere das Vorkommen von Polymorphismen in der Haarnadel, lassen sich durch die Aufzeichnung einer Schmelzkurve detektieren.

Ein bevorzugtes Primer-/Sondensystem umfasst :
a) drei Primer, wobei
   - ein Primerpaar 1 und 2 als Vorwärts- und Rückwärtsprimer fungieren und Primer 1 mindestens zwei für das nachzuweisende Nukleinsäuretarget spezifische Sequenzen (TSS 1 und HBS) umfasst und beide eine enzymatische Verlängerung gestatten, und
   - einen Primer 3, der mit dem 5'-Bereich von Primer 1 teilweise oder vollständig identisch ist, ebenfalls enzymatisch verlängerbar ist und Fluorophor 1 trägt, wobei er so konstruiert ist, dass er eine Haarnadel 1 vollständig oder partiell mit sich selbst oder mit komplementären Abschnitten der Primer 1-Sequenz bilden kann,
b) eine Sonde, die am 3'-Ende Fluorophor 2 trägt und die vollständig für die Targetsequenz spezifisch ist, oder die für die Targetsequenz und Primer 1 spezifisch ist.

Gegenstand der Erfindung kann auch ein Primer-/Sondensystem sein, das umfasst:
a) drei Primer, wobei
   - ein Primerpaar 1 und 2 als Vorwärts- und Rückwärtsprimer fungieren und wobei Primer 1 mindestens zwei für das nachzuweisende Nukleinsäuretarget spezifische Sequenzen (TSS) umfasst und beide eine enzymatische Verlängerung gestatten, und
   - Primer 3, der mit dem 5'-Bereich von Primer 1 teilweise oder vollständig identisch ist und ebenfalls enzymatisch verlängerbar ist,
b) zwei Sonden, die jeweils Fluorophor 1 und 2 tragen und die vollständig für die Targetsequenz spezifisch sind, oder die für die Targetsequenz und Primer 1 spezifisch sind.

Gegenstand der Erfindung kann auch ein Primer-/Sondensystem sein, das umfasst:
a) drei Primer, wobei
   - ein Primerpaar 1 und 2 als Vorwärts- und Rückwärtsprimer fungieren und wobei Primer 1 mindestens zwei für das nachzuweisende Nukleinsäuretarget spezifische Sequenzen (TSS) umfasst und beide eine enzymatische Verlängerung gestatten, und
   - Primer 3, der mit dem 5'-Bereich von Primer 1 teilweise oder vollständig identisch ist und ebenfalls enzymatisch verlängerbar ist,
b) zwei Sonden, die jeweils Fluorophor 1 und 2 tragen und die vollständig für die Targetsequenz spezifisch sind, oder die für die Targetsequenz und Primer 1 spezifisch sind.
c) eine Sonde, die vollständig oder teilweise in einer Haarnadel 2 einer oder mehrerer Targetsequenzen bindet und am 3'-Ende so modifiziert wurde, daß sie enzymatisch nicht verlängert werden kann.

Gegenstand der Erfindung ist auch ein Reagenzienkit zum Nachweis von Nukleinsäuren umfassend
- mindestens ein neuartiges Primer-/Sondensystem,
- geeignete Puffer und ggf. weitere Hilfsstoffe.

Puffer und Hilfsstoffe sind dem Fachmann bekannt. Sie umfassen vorzugsweise thermostabile Polymerase, dNTP-Mix, PCR-Puffer und ggf. MgCl₂.

Bei der technischen Realisierung von Nukleinsäureassays für Routine-Untersuchungen sind insbesondere von Bedeutung, der homogene Assay, das Multiplex-Verfahren und die so genannten Mikroarrays (auch Gen- oder Biochips genannt). In einer erfindungsgemäßen Multiplex-PCR mit dem Primer-/Sondensystem bzw. dem Kit lassen sich mit dem oben beschriebenen Verfahren mehrere Primersätze kombinieren, die aufgrund unterschiedlicher Targetbindungssequenzen (TSS) oder HBS die Detektion mehrerer Zielsequenzen in einem Reaktionsansatz erlauben. Qualitative Messungen erfolgen, indem nach einer vorher definierten Anzahl von Amplifikationszyklen geprüft wird, ob die Konzentration der gebildeten Nukleinsäuremoleküle einen bestimmten Schwellwert übersteigt. Für eine Quantifizierung wird diese Konzentration nach jedem Zyklus erfasst und die Anzahl der Zyklen bis zum Erreichen eines bestimmten Schwellwertes bestimmt. Diese Anzahl ist ein Maß für die Konzentration der gesuchten Nukleinsäure in einer Probe.

Mit Hilfe des erfindungsgemäßen Systems und Verfahrens können zur gleichzeitigen Unterscheidung von mehreren Targetsequenzen die Targetsequenzen im Sondenbindungsbereich, im Primerbindungsbereich oder im Stamm der Haarnadel(n) vorzugsweise durch Schmelzkurvenanalyse unterschieden werden.

Darüber hinaus können zur gleichzeitigen Unterscheidung von mehreren Targetsequenzen mehrere Primer-/Sondensysteme eingesetzt werden, die sich mindestens teilweise oder vollständig in den targetsequenzspezifischen Abschnitten und den Emissionswellenlängen der emittierenden Fluorophore unterscheiden, so dass diese durch Mehrfarbenfluoreszenzdetektion differentiell erfasst werden können.

Weiterhin können zum Nachweis von mehreren Targetsequenzen mittels allelspezifischer PCR die targetspezifischen Primer sich im 3'-Bereich unterscheiden und dadurch vollständig oder unvollständig komplementär mit der Targetsequenz in der jeweiligen Probe sein, wodurch sie detektiert oder nicht detektiert werden.

Weiterhin können zum gleichzeitigen Nachweis von mehreren Targetsequenzen unterschiedliche Detektionssysteme eingesetzt werden, wobei die einzusetzenden targetspezifischen Primer Längenpolymorphismen und Fluoreszenzmarkierungen enthalten können.

Darüber hinaus können die Targetsequenzen durch Echtzeitfluoreszenzanalyse, durch Elektrophorese oder durch Hybridisierung ohne Amplifikation der Targetsequenz, durch Hybridisierung nach der Amplifikation der Targetsequenz oder durch Hybridisierung während der Amplifikation der Targetsequenz in Lösung oder an einer festen Phase erfasst werden. Als bevorzugte feste Phasen kommen fluoreszenzkodierte Beadarrays und ortskodierte Biochips zum Einsatz.

Ihre weite Verbreitung verdankt die Real-Time-PCR zwei Eigenschaften, die im Laboralltag nur selten gut zusammenpassen: sie ist schnell und gleichzeitig präzise. Länger als zwei Stunden benötigt kaum ein Real-Time-Cycler, um winzigste DNA-Mengen zu vervielfältigen und zu quantifizieren. Das Ganze funktioniert auch im Hochdurchsatz. Bis zu 384 PCR-Reaktionen gleichzeitig können Geräte durchführen, deren Cyclerblöcke sich mit entsprechenden Mikrotiterplatten bestücken lassen.

Real-Time-Cycler sind zahlreich bekannt. Sie ermöglichen eine Detektion von bis vorzugsweise zu fünf Fluoreszenzfarbstoffen gleichzeitig für Multiplex Anwendungen, besitzen Heizblöcke vorzugsweise im Viererpack für 96er oder 384er-Well Mikrotiterplatten, zeigen schnelle Heiz-/Kühlraten die z.B. 30 PCR-Zyklen in 30 Minuten möglich machen, Quantifizierungssoftware, Schmelzpunktanalyse und vieles mehr. Das erfindungsgemäße System und Verfahren ist auch für Hochdurchsatzlabore geeignet, die ganze Berge von Proben abarbeiten müssen, wodurch bis zu 5000 Analysen pro Tag mit entsprechenden automatisierten Real-Time-PCR-Robotern der neuesten Generation möglich sind. Falls kein Wert auf Real-Time-Quantifizierung gelegt wird und nur Interesse an der Endpunkt-PCR besteht, können bis zu 30.000 Proben in knapp 3 Stunden durch entsprechende PCR-Roboter bewältigt werden.

Mit dem erfindungsgemäßen System kann ein vorgefertigter Real-Time-PCR-Kit bereitgestellt werden, der alle nötigen Ingredenzien enthält. Damit ist das einzelne Pipettieren von Puffer, Nukleotidmix, Reportermolekülen, Polymerasen und Primern nicht notwendig. Für die Quantifizierung nutzt man bei diesem (klassischen) Verfahren der Real-Time-PCR die Schmelzpunktbestimmung der amplifizierten DNA und die Analyse des Schwellenwertes der Fluoreszenzintensität (CT).

Erfindungsgemäß kann die Verwendung eines erfindungsgemäßen Primer-/Sondenystems in einer Vorrichtung erfolgen, welche die Sonden immobilisiert als Fangsonden umfasst, zur simultanen Detektion von Targetsequenzen während der Amplifikation der Targetsequenzen, wobei die zufällig oder systematisch in der Reaktionsumgebung verteilten Messpunkte, an die die Targetsequenzen über die immobilisierten Fangsonden hybridisieren, bildgebend fluoreszenzoptisch erfasst, dekodiert und vermessen werden und die Reaktionsumgebung thermozyklisch steuerbar ist, ggf. mittels Peltierelementen, Luftstrom, Wasserbad, Wärmestrahlung, Induktion oder Mikrowellen.

Die Durchführung von Real-time-Nachweisen kann unter Verwendung der erfindungsgemäßen Sondensysteme aber auch unter Verwendung von anderen Sondensystemen wie z.B. Molecular Beacon, FRET-Sonden, TaqMan, Scorpions, GenePins oder Amplifluor-Primern vorgenommen werden.

Vorteile des erfindungsgemäßen Verfahrens sind:
- Ein Primer, der universell für verschiedene Amplifikate eingesetzt werden kann trägt bereits ein Fluorophor. Dadurch können kostengünstig die Fluorophore der Sonden verschiedener Amplikons detektiert werden (Beispiel 3).
- Die farbstoffmarkierten Sonden und Primer müssen nicht polymorph sein, da die Polymorphismen über variierende Targetbindungssequenzen von unmarkiertem Primer 1 erfaßt werden können. Dadurch ist es möglich, die farbstoffmarkierten Sonden und Primer auf maximale Detektionssensitivität zu optimieren und die Spezifitätsbestimmung für die Identifizierung von Sequenzunterschieden auf die Optimierung nicht markierter Primer zu begrenzen (z.B. Primer 1, Beispiel 3), was den Kostenaufwand bei der Optimierung der PCR-Systeme reduziert.

- Polymorphe DNA-Abschitte können aber auch über preisgünstige unmarkierte Sonden nachgewiesen werden, die in Haarnadel 2 binden und ein sehr effizientes Öffnen von Haarnadel 2 bewirken, so daß Polymorphismen über Schmelzkurvenverläufe sehr spezifisch und damit sicher nachgewiesen werden können.
- Unterschiedliche Amplikons können über preisgünstige, unmarkierte Sonden nachgewiesen werden, die in Haarnadel 1 binden und ein sehr effizientes Öffnen von Haarnadel 1 bewirken, so daß unterschiedliche Amplikons sehr spezifisch und damit sicher nachgewiesen werden können.
- Durch sehr große Haarnadeln 2 wird die Methode sehr variabel einsetzbar, da die Sondenbindungsstellen am Target in optimale Bindungsbereiche gelegt werden können.
- Durch sehr große Haarnadeln 2 wird die Methode sehr variabel einsetzbar, da durch den Einsatz mehrerer Blockadesonden große Sequenzbereiche auf Sequenzveränderungen untersucht werden können, ohne daß man zusätzliche, markierte Sonden benötigt.
- Durch den Einsatz mehrerer Blockadesonden, können auf einfache Weise größere Sequenzbereiche innerhalb der Haarnadel auf Polymorphismen untersucht werden.
- Da ein Fluoreszenzfarbstoff über einen Primer kovalent an das Amplifikat gebunden wird und somit nur eine Sonde eingesetzt werden muß, können höhere Empfindlichkeiten erzielt werden, können über einen größeren Temperaturbereich stabilere Messwerte erzielt werden, was das System robuster als das klassische Zwei-Sonden-System macht.

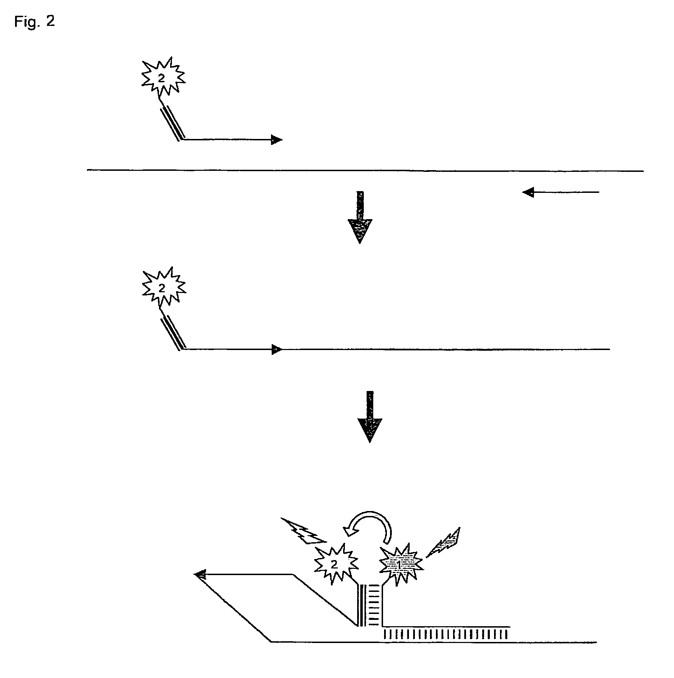

Die Erfindung ist schematisch in Fig. 2 dargestellt.

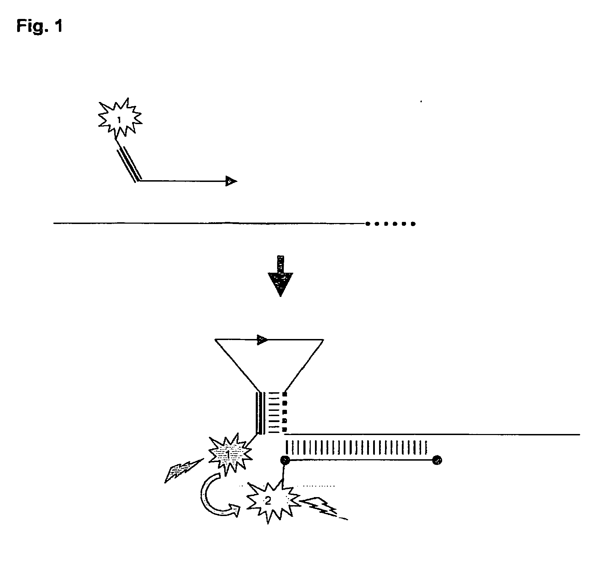

Fig. 1, 3-5 stellen Referenzbeispiele dar.
Nachfolgend wird die Erfindung anhand von Zeichnungen und Ausführungsbeispielen näher erläutert, wobei sie nicht darauf beschränkt werden soll.
**Fig. 1** **- schematische Darstellung des Einsatzes des Primer-/Sondensystems unter Verwendung eines Primers und einer Sonde zur Detektion eines Target-Polymorphismus**
   Primer 1 trägt ein kovalent am 5'-Ende gebundenes Fluorophor 1 sowie eine Targetbindungssequenz 1 und eine Haarnadelbildungssequenz, wobei die Haarnadelbildungssequenz zur Polymorphismuserfassung dient. Primer 1 bindet mit der Targetbindungssequenz 1 seines 3'-Endes an die Targetsequenz in einer 1. Position und wird durch eine DNS-Polymerase unter geeigneten Bedingungen komplementär verlängert. Nach Denaturierung des Doppelstranges bildet das 5'-Ende von Primer 1 über die Haarnadelbildungssequenz (HBS) eine Haarnadel mit der Targetsequenz 2 des Gens, die den Polymorphismus umfasst. Außerdem bindet die Sonde, die Fluorophor 2 am 3'-Ende trägt, an den komplementär zum Target verlängerten Primer unmittelbar anschließend an die gebildete Haarnadel. Durch Bestrahlung des Reaktionsgemisches wird mit Licht definierter Wellenlänge ein von Fluorophor 1 vermittelter FRET ausgelöst und eine Emission von Fluorophor 2 gemessen. Diese Emission korreliert mit der im Reaktionsgemisch befindlichen Menge an Template, die den Polymorphismus umfasst. Zur besseren Unterscheidung von Targetsequenzen mit Polymorphismus und von Targetsequenzen ohne, die sich nur geringfügig in ihrer Targetsequenz 2 unterscheiden wird eine Schmelzkurve erstellt. Sind beide Targetsequenzen im Reaktionsgemisch enthalten, resultieren zwei Peaks in der Darstellung der Schmelzkurve.
**Fig. 2****: Nachweis eines Faktor 2-Polymorphismus**
   Ein Fragment des Faktor 2-Gens wird zwischen Primer 1 und Primer 2 amplifiziert, wobei Primer 1 an seinem 5'-Targetsequenz-unspezifischen Ende ein Fluorophor 2 trägt, welches komplementär zum 3'-Targetsequenz-unspezifischen Ende der Sonde ist. Das 3'-Ende der Sonde trägt Fluorophor 1. Sobald die Sonde an den komplementär zum Target verlängerten Primer 1 bindet, hybridisieren die Targetsequenz-unspezifischen Enden von Primer und Sonde wobei Fluorophor 1 und 2 in räumliche Nähe kommen, so dass FRET stattfindet. Zur Identifizierung von Polymorphismen im Sondenbindungsbereich wird eine Schmelzkurve erzeugt. Es ist aber genauso möglich, zusätzlich eine Sonde 2 einzusetzen, die in den Loop-Bereich hybridisiert und dadurch die Bildung des Loops und damit FRET verhindert (nicht dargestellt). Polymorphismen im Bindungsbereich von Sonde 2 können durch Aufzeichnung der Schmelzkurve von Sonde 2 identifiziert werden.

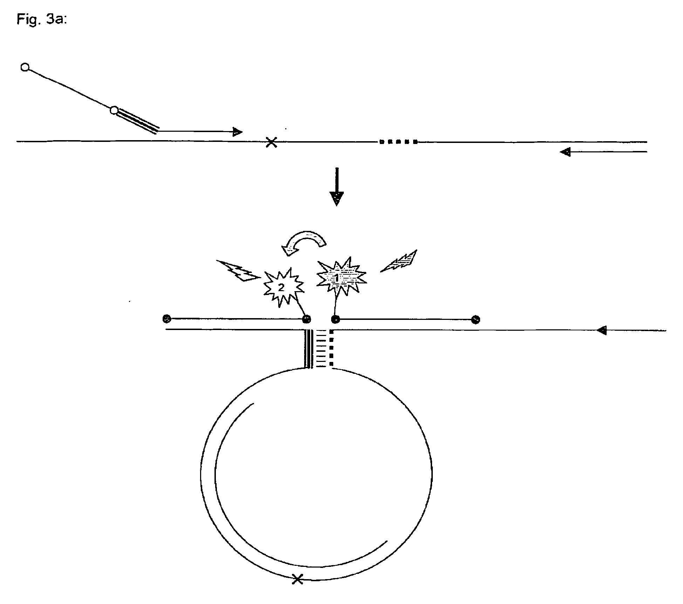

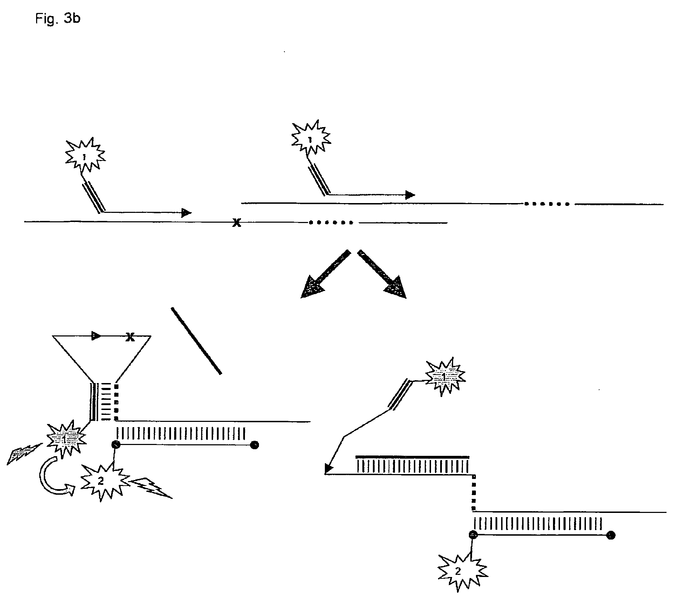

**Fig. 3****: Schematische Darstellung eines Nachweises eines Targetsequenz-Polymorphismus**
   Ein Teil einer Nukleotidsequenz wird zwischen Primer 1 und Primer 2 amplifiziert. Primer 2 ist am 5'-Ende targetsequenzunspezifisch, d.h., ein Teil des Oligonukleotides bindet nicht an das Target. Dieser Teil beinhaltet einen Sequenzabschnitt der komplementär zu einem Sequenzabschnitt weiter in 3'-Richtung auf dem Target ist und mit diesem nach Amplifikation zu einer Haarnadel hybridisieren kann. Weiter in 5'-Richtung auf Primer 2 liegt ein Sequenzabschnitt, der eine Hybridisierungsmöglichkeit für eine Sonde 2 in das Amplifikat einführt. Sonde 1 ist komplementär zum komplementär zum Target verlängerten Primer 2. Nach Hybridisierung zur Haarnadel kommen die Fluorophore der Sonden 1 und 2 so in räumliche Nähe, daß FRET stattfinden kann. Eine Hybridisierung zur Haarnadel wird jedoch in niedrigen Temperaturbereichen verhindert, da dann Sonde 3 in die Haarnadelstruktur binden kann und die Haarnadel nicht geschlossen wird. Die Dehybridisierungstemperatur von Sonde 3 ist abhängig vom Vorkommen des Polymorphismus in der Haarnadel. Durch die Aufnahme einer Schmelzkurve kann der in der Haarnadel liegende Polymorphismus näher bestimmt werden.
   Es ist natürlich ebenso möglich, dass einer der beiden Fluorophore an Primer 1 gebunden vorliegt, wobei in diesem Fall auf die Primer-spezifische Sonde 2 und deren Bindungsbereich in Primer 2 verzichtet werden kann (siehe Fig. 3b)

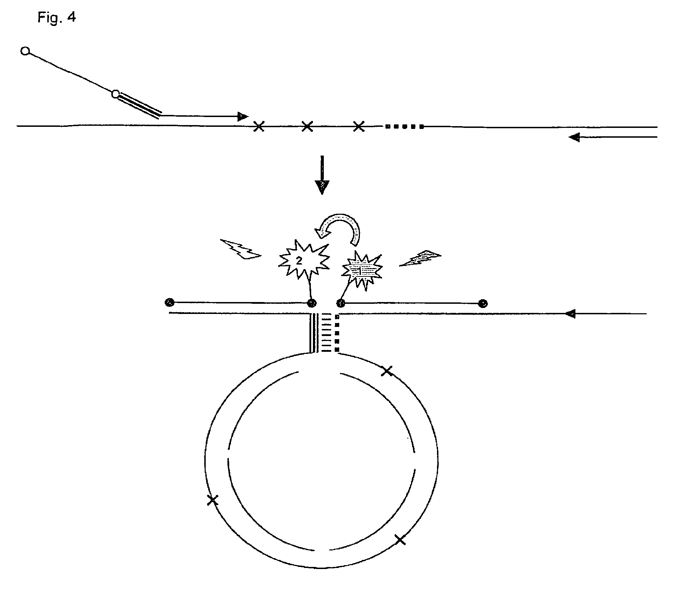

**Fig. 4****: Schematische Darstellung der Charakterisierung von Nukleotidsequenzen durch Schmelzkurvenanalyse mit mehreren Blockadesonden**
   Ein Teil einer Nukleotidsequenz wird zwischen Primer 1 und Primersonde 2 amplifiziert. Die Primersonde 2 ist am 5'-Ende targetsequenzunspezifisch, d.h., ein Teil des Oligonukleotides bindet nicht an das Target. Dieser Teil beinhaltet einen Sequenzabschnitt der komplementär zu einem Sequenzabschnitt weiter in 3'-Richtung auf dem Target ist und mit diesem nach Amplifikation zu einer Haarnadel hybridisieren kann. Weiter in 5'-Richtung auf der Primersonde liegt ein Sequenzabschnitt, der eine Hybridisierungsmöglichkeit für eine Sonde 2 in das Amplifikat einführt. Sonde 1 ist komplementär zu einem Abschnitt des Targets. Nach Hybridisierung zur Haarnadel kommen die Fluorophore der Sonden 1 und 2 so in räumliche Nähe, daß FRET stattfinden kann. Bei niedrigen Temperaturen können die Sonden 3 bis 6 in der Haarnadelstruktur hybridisieren und damit das Schließen der Haarnadel verhindern. Bei Temperaturerhöhung dehybridisieren die Sonden 3 bis 6 entsprechend ihrer spezifischen (unterschiedlichen) Schmelztemperaturen und es kommt zur Ausbildung einer charakteristischen Schmelzkurve. Liegt unter einer Sonde ein Polymorphismus vor, ändert sich das Schmelzkurvenbild typisch für diesen Fall.

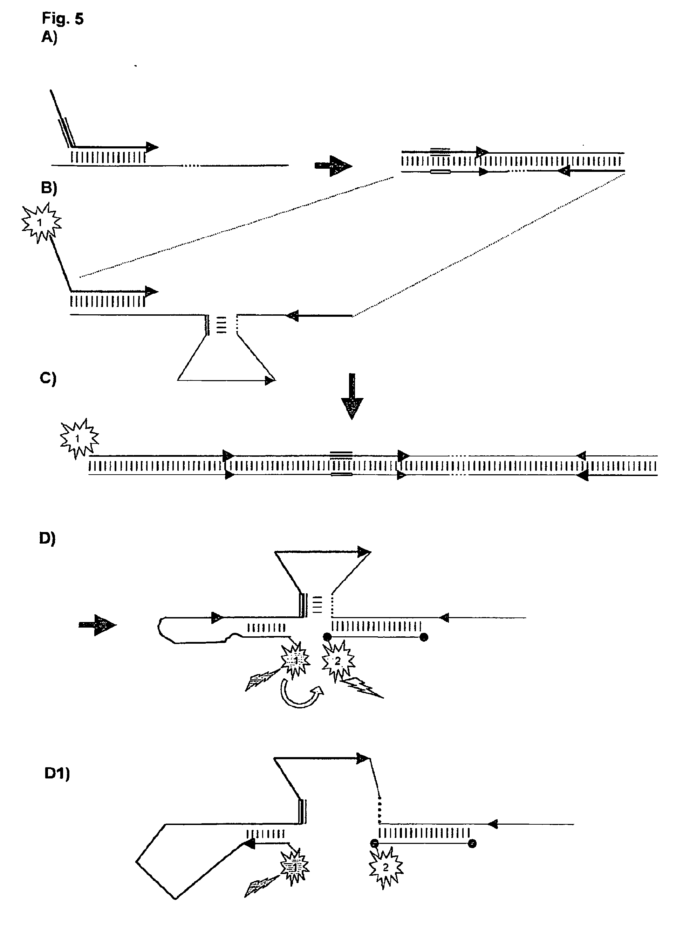

**Fig. 5** **- schematische Darstellung des Einsatzes des Primer-/Sondensystems unter Verwendung dreier Primer und einer Sonde zum Real-time-Nachweis und zum elektrophoretischen Nachweis von Polymorphismen**
   **(A)** Primer 1 umfasst zwei Targetbindungssequenzen 1 und 2 sowie ein 5'-Ende, welches der Sequenz von Primer 3 entspricht. Primer 1 bindet mit der Targetbindungssequenz 1 seines 3'-Endes an die Targetsequenz des zu detektierenden Gens und wird durch eine DNS-Polymerase unter geeigneten Bedingungen komplementär zum Target verlängert. Nach Denaturierung des Doppelstranges bindet Primer 2 an den verlängerten Primer 1 und wird komplementär dazu verlängert.
   **(B)** Der resultierende Doppelstrang wird denaturiert, so dass **(C)** Primer 3 mit Fluorophor 1 am 5'-Ende an das komplementäre Ende von Primer 1 bindet und verlängert wird.
   **(D)** Der resultierende Doppelstrang wird denaturiert, so dass bei der Rehybridisierung sich eine Haarnadel 1 zwischen Targetbindungssequenz 2 und Targetsequenz 2 ausbildet. Außerdem bildet sich zwischen dem 5'-Ende der Primer 3-Sequenz und Primer 1 eine Haarnadel 2 aus. Die Sonde, die eine Markierung mit Fluorophor 2 am 3'-Ende trägt, bindet an den komplementär zum Target verlängerten Primer 3 unmittelbar anschließend an die Haarnadel 1.
   **(D1)** Zur selektiven Amplifikation unterschiedlicher Targetsequenzen unterscheidet sich Primer 1 in der Targetbindungssequenz 1 und durch einen Längenmarker zwischen Primerbindungssequenz 1 am 3'-Ende sowie Primersequenz 3 an seinem 5'-Ende von anderen Primern 1 für andere Targetsequenzen im Multiplex-Ansatz.
   Durch Bestrahlung des Reaktionsgemisches mit Licht definierter Wellenlänge wird ein über Fluorophor 1 vermittelter FRET ausgelöst und eine Emission von Fluorophor 2 bei definierter Wellenlänge gemessen. Diese Emission korreliert mit der im Reaktionsgemisch befindlichen Menge an Template, die den Polymorphismus umfasst. Zur besseren Unterscheidung von Targetsequenzen mit Polymorphismus und von Targetsequenzen ohne, die sich nur geringfügig in ihrer Targetsequenz 2 unterscheiden wird eine Schmelzkurve erstellt. Sind beide Targetsequenzen im Reaktionsgemisch enthalten, resultieren zwei Peaks in der Darstellung der 1. Ableitung der Schmelzkurve. Auf Grund unterschiedlicher Längenmarker in unterschiedlichen Primern 1 können die Amplifikate zusätzlich auch durch Gelelktrophorese oder Massenspektroskopie detektiert bzw. unterschieden werden.

### Beispiel 1

### Nachweis des Erregers Herpes-simplex-Virus in einer Probe

Ein Volumen von 5 µl aus Abstrichen von Lippenbläschen gereinigter Proben-DNS wird zu 15 µl Master-Mix folgender Zusammensetzung gegeben:
Primer 1 400 nM
Primer 2 300 nM
Sonde 100 nM
in Taq-DNS-Polymerase, Nukleotide, Magnesiumchlorid, Tris/HCI-Puffer und Tween 20 gelöst.

Der Reaktionsansatz wird in eine LightCycler-Kapillare pipettiert und anschließend in den LightCycler überführt und thermocyclisch amplifiziert. Das Verfahren ist schematisch in Fig. 1 dargestellt. Primer 1 trägt ein kovalent gebundenes Fluorescein am 4. Nukleotid vom 3'-Ende aus gezählt, bindet mit seiner Targetbindungssequenz 1 am 3'-Ende an die Targetsequenz des *Herpes simplex*-Virus-Genoms und wird durch eine DNS-Polymerase unter geeigneten Bedingungen komplementär zum Target verlängert. Nach Denaturierung des Doppelstranges kann die Sonde, die eine Rhodamin-Markierung am 3'-Ende trägt an den komplementär zum Target verlängerten Primer hybridisieren. Durch Bestrahlung des Reaktionsgemisches mit Licht einer Wellenlänge von 480 nm wird ein über Fluorescein vermittelter FRET ausgelöst und die Emission bei 560 nm korreliert mit der im Reaktionsgemisch befindlichen Menge an Template.
Nach Abschluß der Amplifikation wird für alle Proben, die ein ausreichend hohes Meßsignal aufweisen, eine Schmelzkurve aufgezeichnet.
Sequenzen G an Position 2 (grau hinterlegt) trägt kovalent ein FITC-Molekül.
Primer 2: 5'-CAT CAC CGA CCC GGA GAG GGA C-3' (SEQ ID No: 2)
Sonde: 5'-GCAACTCTCTCAGGGCCAGGCGG-3'-Rhodamin (SEQ ID No: 3)

### Beispiel 2:

### Nachweis eines Faktor 2-Polymorphismus

Ein Volumen von 5 µl aus Patientenblut gereinigter Proben-DNS oder zur Reamplifkation bestimmtes, vorverdünntes Amplifikat wird zu 15 µl Master-Mix folgender Zusammensetzung gegeben:
Primer 1 (FITC-modifiziert) 300 nM
Primer 2 300 nM
Sonde 100 nM
in Taq-DNS-Polymerase, Nukleotide, Magnesiumchlorid, Tris/HCI-Puffer und Tween 20 gelöst.
Der Reaktionsansatz wird in eine LightCycler-Kapillare pipettiert und anschließend in den LightCycler überführt und thermocyclisch amplifiziert. Das Verfahren ist schematisch in Fig. 2 dargestellt. Primer 1 trägt ein kovalent am 5'-Ende gebundenes Cy5 sowie eine Targetbindungssequenz im 3'-Bereich und eine Sondenbindungssequenz im 5'-Bereich. Mit der Targetbindungssequenz bindet Primer 1 an das Target und wird durch eine DNS-Polymerase unter geeigneten Bedingungen komplementär zum Target, dem Faktor 2-Gen, verlängert. Nach Denaturierung des Doppelstranges bildet das 5'-Ende von Primer 1 einen haarnadelartigen Loopmit dem Target-unspezifschen 3'-Bereich der Sonde, deren 3'-Ende durch eine Fluoresceinmarkierung gegen Polymeraseverlängerung blockiert wurde. Durch Bestrahlung des Reaktionsgemisches mit Licht einer Wellenlänge von 480 nm wird ein über Fluorescein vermittelter FRET ausgelöst und eine Emission von Cy5 bei 640 nm gemessen. Diese Emission korreliert mit der im Reaktionsgemisch befindlichen Menge an Template, die den Polymorphismus umfaßt. Zur besseren Unterscheidung von Targetsequenzen mit Polymorphismus und von Targetsequenzen ohne, die sich nur geringfügig in ihrer Targetsequenz 2 unterscheiden wird eine Schmelzkurve erstellt. Sind beide Targetsequenzen im Reaktionsgemisch enthalten, resultieren zwei Peaks in der Darstellung der Schmelzkurve. Der Sequenzpolymorphismus wird bevorzugt in den Target-spezifischen Bereich der Sonde detektiert. Bei vorliegen einer Mutation der Targetsequenz im Sondenbindungsbereich kommt es zu einer Fehlpaarung bei der Sondenbindung, so daß sich die Schmelztemperatur der Sonde verändert.
Nach Abschluß der Amplifikation wird für alle Proben, die ein ausreichend hohes Meßsignal aufweisen, eine Schmelzkurve aufgezeichnet, um Veränderungen des Schmelzverhaltens der Sonde und damit von etwaigen Mutationen zu finden..
Sequenzen
Primer 1: Cy5-5'-GTCGTCGGCCTGGAACCAATCCCGTGAAAG-3' (SEQ ID No: 4)
Primer 2: 5'-CCA GGT GGT GGA TTC TTA AGT C-3' (SEQ ID No: 5)
Sonde: 5'-CATTGAGGCTCGCTGAGAGTGCCGACGAC-3'-FITC (SEQ ID No: 6)

### Beispiel 3:

### Nachweis eines Targetsequenz-Polymorphismus

Ein Volumen von 5 µl gereinigter Proben-DNS wird zu 15 µl Master-Mix folgender Zusammensetzung gegeben:

| | |
|---|---|
| Primer 1 | 300 nM |
| Primer 2 | 300 nM |
| Sonde 1 (FITC-modifiziert) | 400 nM |
| Sonde 2 (LC-Red-640-modifiziert) | 400 nM |
| Sonde 3 | 500 nM |

in Taq-DNS-Polymerase, Nukleotide, Magnesiumchlorid, Tris/HCI-Puffer und Tween 20 gelöst.

Der Reaktionsansatz wird in eine LightCycler-Kapillare pipettiert und anschließend in den LightCycler überführt und thermocyclisch amplifiziert. Das Verfahren ist schematisch in Fig. 3 dargestellt. Beide Primer sind nicht mit Fluoreszenzfarbstoffen markiert. Primer 2 dient dabei einerseits als Primer (Sequenzbereich am 3'-Ende) und andererseits dazu, die komplementäre Sequenz zur Haarnadelstammbildung sowie zum Binden der Sonde 2 (FRET-Akzeptor) in das Amplifikat einzuführen. Das durch PCR entstehende Amplifikat bildet eine 20 Basen lange Haarnadelstruktur an der die Sonde 3 hybridisieren kann. Dabei überdeckt Sonde 3 eine zu detektierende Mutation (x).
Außerdem binden eine mit einem Fluoreszenzfarbstoff markierte Sonde 1 (FRET-Donor, z.B. Fluorescein) in direkter Nähe (vorzugsweise 0 bis 10 Basen Abstand zur ersten Base des Haarnadelstammes) zum Stamm der Haarnadel wie auch Sonde 2 (FRET-Akzeptor, z.B. LC-Red-640) deren Fluroeszenzmarkierung am entgegengesetzten Ende der Sonde 1 liegt (z.B. Sonde 1 5'-markiert, Sonde 2 3'-markiert). Bei geschlossener Haarnadel kommen die mit Fluoreszenzfarbstoffen markierten Sonden in unmittelbare Nähe zueinander und es kann bei Anregung mit einer geeigneten Wellenlänge ein Fluoreszenzsignal des FRET-Akzeptors detektiert werden.
Durch die ebenfalls im Reaktionsansatz vorhandene Sonde 3 wird bei niedrigen Temperaturen eine Öffnung der Haarnadel erzwungen, da Sonde 3 so in der Haarnadelstruktur hybridisieren kann, daß das Schließen der Haarnadel verhindert wird. Erst nach Erhöhung der Temperatur (z.B. bei Aufnahme einer Schmelzkurve) dehybridisiert Sonde 3 und die Haarnadel schließt sich (Erhöhung des Fluoreszenzsignals). Beim Vorhandensein eines Basenaustausches im Haarnadelbereich (nachzuweisende Mutation) dehybridisiert die Sonde entsprechend früher und führt zu einem veränderten Signalverlauf. Die Veränderungen können mit einer Schmelzkurvenanalyse leicht detektiert werden (Doppelpeak beim Vorhandensein von Wildtyp- und Mutationstemplate, heterozygote Konstellation).

### Sequenzen

| | |
|---|---|
| Primer 1: | 3'-CTGAATTCTTAGGTGGTGGACC-5' (SEQ ID No: 7) |
| Primer 2 | 5'-GATCCAAGTCCAAACGTCAACCCATCTGACTACGCTGAGTGTTCCCAATAAAAGTGACTC-3' (SEQ ID No: 8) |
| Sonde 1 | (Fluorescein-modifiziert):FluoresCein-5_{'}-TAGTCAGATGGGTTGACGTTTGGACTTGGATC-3' (SEQ ID No: 9) |
| Sonde 2 (LC-Red-640-modifiziert): | 5'-CCATGAATAGCACTGGGAGCATTGAGGCT-3'-LC-Red640 (SEQ ID No: 10) |
| Sonde 3: | 5'-GTCACTTTTATTGGGAAC-3' (SEQ ID No: 11) |

### Beispiel 4:

### Charakterisierung von Nukleotidsequenzen durch Schmelzkurvenanalyse

Ein Volumen von 5 µl gereinigter Proben-DNS zur Reamplifikation bestimmtes Amplifikat wird zu 15 µl Master-Mix folgender Zusammensetzung gegeben:

| | |
|---|---|
| Primer 1 | 300 nM |
| Primer 2 | 300 nM |
| Sonde 1 (Fluorescein-modifiziert) | 400 nM |
| Sonde 2 (LC-Red-640-modifiziert) | 400 nM |
| Sonde 3 bis 6 | 500 nM |

in Taq-DNS-Polymerase, Nukleotide, Magnesiumchlorid, Tris/HCI-Puffer und Tween 20 gelöst.
Der Reaktionsansatz wird in eine LightCycler-Kapillare pipettiert und anschließend in den LightCycler überführt und thermocyclisch amplifiziert. Das Verfahren ist schematisch in Fig. x dargestellt. Beide Primer sind nicht mit Fluoreszenzfarbstoffen markiert. Primersonde 2 dient dabei einerseits als Primer (Sequenzbereich am 3'-Ende) und andererseits dazu, die komplementäre Sequenz zur Haarnadelstammbildung sowie zum Binden der Sonde 2 (FRET-Akzeptor) in das Amplifikat einzuführen. Das durch PCR entstehende Amplifikat bildet eine 66 Basen lange Haarnadelstruktur an der die Sonden 3 bis 6 hybridisieren können. Bei vollständiger Hybridisierung der Sonden 3 bis 6 wird eine Hybridisierung des Haarnadelstammes verhindert und damit kann bei Anregung mit Licht der Wellenlänge 480 nm kein FRET stattfinden. Bei einer Temperaturerhöhung im Verlaufe der Aufnahme einer Schmelzkurve dehybridisieren die Sonden in definierter Reihenfolge und in Abhängigkeit der Sequenz des gebildeten Amplifikates. Sind im Sondenbindungsbereich Sequenzveränderungen im Vergleich zur Wildtypsequenz vorhanden, wird eine veränderte Schmelzkurve erhalten, die Rückschlüsse auf den Sequenzbereich, der sich verändert hat, zuläßt.

### Sequenzen

| | |
|---|---|
| Primer 1: | 3'-CTGAATTCTTAGGTGGTGGACC-5' (SEQ ID No: 12) |
| Primersonde 2: | 5'-GATCCAAGTCCAAACGTCAACCCATCTGACTACTGGCGCTGAGTTTGTCGTTGTTCCGTC-3' (SEQ ID No: 13) |
| Sonde 1 (Fluorescein-modifiziert): | 5'-Fluorescein-TAGTCAGATGGGTTGACGTTTGGACTTGGATC-3' (SEQ ID No: 14) |
| Sonde 2 | (LC-Red-640-modifiziert):5'-CCATGAATAGCACTGGGAGCATTGAGGCT-LC-Red640-3' (SEQ ID No: 15) |
| Sonde 3: | 5'-GACGGAACAACGACAA-3' (SEQ ID No: 16) |
| Sonde 4: | 5'-GGTCACTTGGATTGG-3' (SEQ ID No: 17) |
| Sonde 5: | 5'-CAGCGGCAGCGACAA-3' (SEQ ID No: 18) |
| Sonde 6: | 5'-GTCACTTTTATTGGGAAC-3' (SEQ ID No: 19) |

### Beispiel 5:

### Simultaner Nachweis von Herpes simplex DNS und interner Standard in einer Probe

Ein Volumen von 5 µl aus Patientenblut gereinigter Proben-DNS wird zu 15 µl Master-Mix folgender Zusammensetzung gegeben:

| | |
|---|---|
| Primer 1a | 10 nM |
| Primer 1b | 10 nM |
| Primer 2 | 300 nM |
| Primer 3 (FITC-modifiziert) | 300 nM |
| Sonde 1 | 100 nM |
| Sonde 2 | 100 nM |
| Interner Standard | 100 fM |

in Taq-DNS-Polymerase, Nukleotide, Magnesiumchlorid, Tris/HCl-Puffer und Tween 20 gelöst.
Der Reaktionsansatz wird in eine LightCycler-Kapillare pipettiert und anschließend in den LightCycler überführt und thermocyclisch amplifiziert. Das Verfahren ist schematisch in Fig. 3 dargestellt. Nach Abschluß der Amplifikation wird für alle Proben, die ein ausreichend hohes Meßsignal aufweisen, eine Schmelzkurve aufgezeichnet, um Amplifikat des internen Standards und, bei HSV-positiven Proben, HSV-Amplifikat zu detektieren.

### Sequenzen

| | |
|---|---|
| Primer 1a: | |
| Primer 1b: | |
| Primer 2: | 5'-GGG CCA GGC GCT TGT TGG TGT A-3' (SEQ ID No: 22) |
| Primer 3: | FITC-5'-actgcacgctcacacggacg-3' (SEQ ID No: 23) |
| Sonde 1: | 5'-tcagttcggcggtgaggaca-3'-Rhodamin (SEQ ID No: 24) |
| | |

**Legende zu den Abbildungen ...**

| | | | |
|---|---|---|---|
| 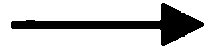 | Primer | 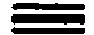 | HBS |
| 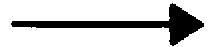 | Komplementär zum Primer | 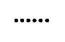 | Targetsequenz 2 |
| 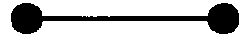 | Sonde | 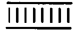 | Hybridisierter Nukleinsäuredoppelstrang |
| 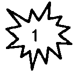 | Fluorophor 1 | | |
| 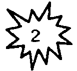 | Fluorophor 2 | 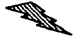 | Anregung |
| 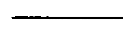 | Target | 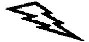 | Emission |
| 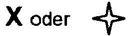 | Polymorphismus im Target | 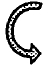 | FRET |
| 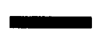 | Sonde 2 | | |

## Patentansprüche

1. Verfahren zum Echtzeit-Nachweis von Nukleinsäuretargets als Produkte einer Nukleinsäure-Amplifikationsreaktion mittels eines Primer/Sondensystems, **dadurch** charakterisiert, dass es folgende Schritte umfasst:
a) Einsatz von mindestens einem Primer der am 3'-Ende eine enzymatische Verlängerung komplementär zum Target gestattet, wobei der mindestens eine Primer
- mindestens eine für die zu amplifizierende Nukleinsäure targetspezifische Sequenz (TSS) aufweist,
- sowie eine Haarnadelbildungssequenz (HBS) umfasst, wobei sich die HBS am 5'-Ende des Primers befindet, targetunspezifisch ist und komplementär zum 3'-Ende einer Sonde 2 ausgebildet ist;
b) Einführung mindestens eines Fluorophor 1 durch kovalente Bindung an das 5'-Ende des mindestens einen Primers aus Schritt a);
c) Enzymatische Verlängerung des Primers am 3'-Ende;
d) Einsatz von mindestens einer Sonde 2, die mindestens am 3'-Ende ein Fluorophor 2 trägt, wobei die mindestens eine Sonde 2 mit ihrem 5'-Bereich an den Targetbereich des am 3'-Ende verlängerten Primer hybridisiert und mit ihrem 3'-Ende an die HBS am targetunspezifischen 5'-Bereich des verlängerten Primers hybridisiert, sodass Fluorophor 1 und Fluophor 2 so in räumliche Nähe gelangen, dass ein Fluoreszenzresonanzenergietransfer zwischen den Fluorophoren 1 und 2 möglich ist;
e) Ausbildung von mindestens einer intermolekularen Haarnadel durch Hybridisierung der HBS des gemäß Schritt c) verlängerten mindestens einen Primers mit dem zur HBS komplementären Abschnitt der Sonde 2.
f) Anregung eines der beiden Fluorophore durch Licht geeigneter Wellenlänge, so dass zwischen den Fluorophoren 1 und 2 ein Fluoreszenzresonanzenergietransfer stattfindet.
g) Fluoreszenzoptische Detektion der Fluoreszenz.

2. Primer-/Sondensystem zum Nachweis von Nukleinsäuren umfassend:
a) einen Primer, der mindestens eine für das nachzuweisende Nukleinsäuretarget spezifische Sequenz umfasst und an seinem 3'-Ende eine enzymatische Target-spezifische Verlängerung gestattet, wobei dieser Primer an seinem 5'-Ende mit dem Fluorophor 1 markiert ist,
b) eine Sonde 2, die an ihrem Target-unspezifischen 3'-Ende ein Fluorophor 2 trägt und die mit ihrem 5'-Bereich an den enzymatisch verlängerten Targetspezifischen Bereich des Primers aus a) bindet;
**dadurch gekennzeichnet, dass**
das 5'-Ende des Primers Target-unspezifisch und komplementär zum 3'-Ende der Sonde ausgebildet ist.

3. Reagenzienkit zum Nachweis von Nukleinsäuren umfassend
1. mindestens ein Primer/Sondensystem gemäß Anspruch 2;
2. geeignete Puffer und gegebenenfalls weitere Hilfsstoffe.

4. Verwendung eines Primer-/Sondenystems nach Anspruch 2 oder eines Reagenzienkits gemäß Anspruch 3 zur gleichzeitigen Unterscheidung von mehreren Targetsequenzen, wobei mehrere Primer-/Sondensysteme eingesetzt werden, die sich mindestens teilweise oder vollständig in den targetsequenzspezifischen Abschnitten und den Emissionswellenlängen der emittierenden Fluorophore unterscheiden, so dass diese durch Mehrfarbenfluoreszenzdetektion differentiell erfasst werden können.

5. Verwendung eines Primer-/Sondenystems nach Anspruch 2 oder eines Reagenzienkits gemäß Anspruch 3 zum Nachweis von mehreren Targetsequenzen, wobei die targetspezifischen Primer sich im 3'-Bereich unterscheiden und **dadurch** vollständig oder unvollständig komplementär mit der Targetsequenz in der jeweiligen Probe sind und **dadurch** detektiert oder nicht detektiert werden.

6. Verwendung eines Primer-/Sondenystems nach Anspruch 2 oder eines Reagenzienkits gemäß Anspruch 3, in einem Mikroarray, welches die Sonden als Fangsonden oder eine Teilmenge eines Primers als Fangprimer immobilisiert umfasst.

7. Verfahren nach Anspruch 1, **dadurch gekennzeichnet, dass** zur gleichzeitigen Unterscheidung von mehreren Targetsequenzen, die Targetsequenzen im Sondenbindungsbereich, im Primerbindungsbereich, im Stamm der Haarnadel(n) durch Schmelzkurvenanalyse oder durch den Einsatz mehrerer Fluorophore oder Längenmarker unterschieden werden.

## Claims

1. A method for the real-time detection of nucleic acid targets as products of a nucleic acid amplification reaction by means of a primer/probe system, **characterized in that** it comprises the following steps:
a) using at least one primer which allows for at the 3' end an enzymatic extension complementary to the target, wherein the at least one primer
- has at least one target-specific sequence (TSS) that is specific for the nucleic acid to be amplified,
- and comprises at least one hairpin-forming sequence (HFS), wherein the HFS is located at the 5' end of the primer, is target-unspecific and is complementary to the 3' end of a probe 2;
b) introducing at least one fluorophore 1 by covalent bonding at the 5' end of the at least one primer from step a) ;
c) enzymatically extending the primer at the 3' end;
d) using at least one probe 2, which has a fluorophore 2 at least at the 3' end, wherein the at least one probe 2 hybridizes with its 5' region to the target region of the primer extended at the 3' end and hybridizes with its 3' end to the HFS on the target-unspecific 5' region of the extended primer, so that fluorophore 1 and fluorophore 2 enter into spatial proximity to one another such that a fluorescence resonance energy transfer between the fluorophores 1 and 2 is possible;
e) forming at least one intermolecular hairpin through hybridization of the HFS of the at least one primer extended according to step c) with the portion of the probe 2 that is complementary to the HFS,
f) exciting one of the two fluorophores by light of suitable wavelength so that a fluorescence resonance energy transfer takes place between the fluorophores 1 and 2,
g) detecting the fluorescence by means of optical fluorescence imaging.

2. A primer/probe system for detecting nucleic acids, comprising:
a) a primer which comprises at least one sequence specific for the nucleic acid target to be detected and which at its 3' end allows for an enzymatic, target-specific extension, wherein this primer is labelled at its 5' end with the fluorophore 1,
b) a probe 2 which has a fluorophore 2 at its target-unspecific 3' end and which bonds with its 5' region to the enzymatically extended target-specific region of the primer from a);
**characterized in that**
the 5' end of the primer is target-unspecific and is complementary to the 3' end of the probe.

3. A reagent kit for detecting nucleic acids, comprising
1. at least one primer/probe system according to claim 2;
2. suitable buffers and optionally further auxiliaries.

4. The use of a primer/probe system according to claim 2 or of a reagent kit according to claim 3 for simultaneously distinguishing a plurality of target sequences, wherein use is made of a plurality of primer/probe systems which differ from one another at least partially or entirely in the target-sequence-specific portions and in the emission wavelengths of the emitting fluorophores, so that they can be detected differentially by multicolour fluorescence detection.

5. The use of a primer/probe system according to claim 2 or of a reagent kit according to claim 3 for detecting a plurality of target sequences, wherein the target-specific primers differ in the 3' region and are thus entirely or non-entirely complementary to the target sequence in the respective sample and as a result are detected or not detected.

6. The use of a primer/probe system according to claim 2 or of a reagent kit according to claim 3 in a microarray which comprises the probes as capture probes or a subset of a primer as a capture primer.

7. The method according to claim 1, **characterized in that**, in order simultaneously to distinguish a plurality of target sequences, the target sequences are distinguished in the probe binding region, in the primer binding region or in the stem of the hairpin(s) by melting curve analysis or by using a plurality of fluorophores or size markers.

## Revendications

1. Procédé de mise en évidence en temps réel d'acides nucléiques cibles en tant que produits d'une réaction d'amplification d'acide nucléique au moyen d'un système de sondes et d'amorces, **caractérisé en ce qu'**il comprend les étapes suivantes :
a) Utilisation d'au moins une amorce qui permet un prolongement enzymatique complémentaire de la cible à l'extrémité 3', l'amorce au moins au nombre de un
• présentant au moins une séquence spécifique de la cible (TSS) pour l'acide nucléique à amplifier,
• ainsi qu'une séquence de formation d'épingle à cheveux (HBS), la HBS étant située à l'extrémité 5' de l'amorce, étant non spécifique de la cible et étant constituée de façon complémentaire de l'extrémité 3' d'une sonde 2 ;
b) Introduction d'au moins un fluorophore 1 par liaison covalente à l'extrémité 5' de l'amorce issue de l'étape a) ;
c) Prolongement enzymatique de l'amorce à l'extrémité 3' ;
d) Utilisation d'au moins une sonde 2 qui porte un fluorophore 2 au moins à l'extrémité 3', la sonde 2 au moins au nombre de un étant hybridée avec sa zone 5' sur la zone cible de l'amorce prolongée à l'extrémité 3' et étant hybridée à la HBS avec son extrémité 3' sur la zone 5' non spécifique de la cible de l'amorce prolongée, de telle sorte que le fluorophore 1 et le fluorophore 2 se rapprochent dans l'espace au point qu'un transfert d'énergie de fluorescence par résonance entre les fluorophores 1 et 2 est possible ;
e) Formation d'au moins une épingle à cheveux intermoléculaire par hybridation de la HBS de l'amorce au moins au nombre de un prolongée selon l'étape c) avec le segment de la sonde 2 complémentaire de la HBS.
f) Excitation d'un des deux fluorophores par une lumière de longueur d'onde appropriée de telle sorte qu'il se produit un transfert d'énergie de résonance par fluorescence entre les fluorophores 1 et 2.
g) Détection optique par fluorescence de la fluorescence.

2. Système de sondes et d'amorces pour la mise en évidence d'acides nucléiques, comprenant :
a) une amorce qui comprend au moins une séquence spécifique pour l'acide nucléique cible à mettre en évidence et qui permet à son extrémité 3' un prolongement enzymatique spécifique de la cible, cette amorce étant marquée à son extrémité 5' avec le fluorophore 1,
b) une sonde 2 qui porte un fluorophore 2 à son extrémité 3' non spécifique de la cible et qui, avec sa zone 5', fait la liaison avec la zone spécifique de la cible prolongée enzymatiquement de l'amorce issue de a) ;
**caractérisé en ce que**
l'extrémité 5' de l'amorce est constituée de façon non spécifique de la cible et complémentaire de l'extrémité 3' de la sonde.

3. Trousse de réactifs pour la mise en évidence d'acides nucléiques, comprenant
1. au moins un système de sondes et d'amorces selon la revendication 2 ;
2. des tampons appropriés et éventuellement d'autres adjuvants.

4. Utilisation d'un système de sondes et d'amorces selon la revendication 2 ou d'une trousse de réactifs selon la revendication 3 pour la distinction simultanée de plusieurs séquences cibles, plusieurs systèmes de sondes et d'amorces étant utilisés qui se distinguent au moins partiellement ou complètement dans les segments spécifiques des séquences cibles et dans les longueurs d'onde d'émission des fluorophores émetteurs de telle sorte que ceux-ci peuvent être saisis de façon différentielle par la détection de fluorescence multicolore.

5. Utilisation d'un système de sondes et d'amorces selon la revendication 2 ou d'une trousse de réactifs selon la revendication 3 pour la mise en évidence de plusieurs séquences cibles, les amorces spécifiques des cibles se distinguant dans la zone 3' et étant totalement ou non totalement complémentaires de la séquence cible dans l'échantillon respectif et étant de ce fait détectées ou non détectées.

6. Utilisation d'un système de sondes et d'amorces selon la revendication 2 ou d'une trousse de réactifs selon la revendication 3, dans un micro-réseau, qui comprend sous forme immobilisée les sondes en tant que sondes de capture ou une quantité partielle d'une amorce en tant qu'amorce de capture.

7. Procédé selon la revendication 1, **caractérisé en ce que**, pour la distinction simultanée de plusieurs séquences cibles, les séquences cibles sont distinguées dans la zone de liaison de sondes, dans la zone de liaison d'amorces, dans le tronc de/des épingle(s) à cheveux par l'analyse de courbes de fusion ou par l'utilisation de plusieurs fluorophores ou marqueurs de longueur.


### SEQUENCE LISTING

<110> Attomol GmbH
<120> Verfahren und Sonden/Primersystem zum "real time" Nachweis eines Nukleinsäuretargets
<130> P471207 PCT1
<150> DE 10 2007 031 137.2-41
   <151> 2007-06-29
<160> 25
<170> PatentIn version 3.3
<210> 1
   <211> 26
   <212> DNA
   <213> artificial sequence
<220>
   <223> primer
<400> 1
   tgaagggcca ggcgcttgtt ggtgta 26
<210> 2
   <211> 22
   <212> DNA
   <213> artificial sequence
<220>
   <223> primer
<400> 2
   catcaccgac ccggagaggg ac 22
<210> 3
   <211> 23
   <212> DNA
   <213> artificial sequence
<220>
   <223> sonde
<400> 3
   gcaactctct cagggccagg cgg 23
<210> 4
   <211> 30
   <212> DNA
   <213> artificial sequence
<220>
   <223> primer
<400> 4
   gtcgtcggcc tggaaccaat cccgtgaaag 30
<210> 5
   <211> 22
   <212> DNA
   <213> artificial sequence
<220>
   <223> primer
<400> 5
   ccaggtggtg gattcttaag tc 22
<210> 6
   <211> 29
   <212> DNA
   <213> artificial sequence
<220>
   <223> sonde
<400> 6
   cattgaggct cgctgagagt gccgacgac 29
<210> 7
   <211> 22
   <212> DNA
   <213> artificial sequence
<220>
   <223> primer
<400> 7
   ctgaattctt aggtggtgga cc 22
<210> 8
   <211> 60
   <212> DNA
   <213> artificial sequence
<220>
   <223> primer
<400> 8
   gatccaagtc caaacgtcaa cccatctgac tacgctgagt gttcccaata aaagtgactc 60
<210> 9
   <211> 32
   <212> DNA
   <213> artificial sequence
<220>
   <223> sonde
<400> 9
   tagtcagatg ggttgacgtt tggacttgga tc 32
<210> 10
   <211> 29
   <212> DNA
   <213> artificial sequence
<220>
   <223> sonde
<400> 10
   ccatgaatag cactgggagc attgaggct 29
<210> 11
   <211> 18
   <212> DNA
   <213> artificial sequence
<220>
   <223> sonde
<400> 11
   gtcactttta ttgggaac 18
<210> 12
   <211> 22
   <212> DNA
   <213> artificial sequence
<220>
   <223> primer
<400> 12
   ctgaattctt aggtggtgga cc 22
<210> 13
   <211> 60
   <212> DNA
   <213> artificial sequence
<220>
   <223> primersonde
<400> 13
   gatccaagtc caaacgtcaa cccatctgac tactggcgct gagtttgtcg ttgttccgtc 60
<210> 14
   <211> 32
   <212> DNA
   <213> artificial sequence
<220>
   <223> sonde
<400> 14
   tagtcagatg ggttgacgtt tggacttgga tc 32
<210> 15
   <211> 29
   <212> DNA
   <213> artificial sequence
<220>
   <223> sonde
<400> 15
   ccatgaatag cactgggagc attgaggct 29
<210> 16
   <211> 16
   <212> DNA
   <213> artificial sequence
<220>
   <223> sonde
<400> 16
   gacggaacaa cgacaa 16
<210> 17
   <211> 15
   <212> DNA
   <213> artificial sequence
<220>
   <223> sonde
<400> 17
   ggtcacttgg attgg 15
<210> 18
   <211> 15
   <212> DNA
   <213> artificial sequence
<220>
   <223> sonde
<400> 18
   cagcggcagc gacaa 15
<210> 19
   <211> 18
   <212> DNA
   <213> artificial sequence
<220>
   <223> sonde
<400> 19
   gtcactttta ttgggaac 18
<210> 20
   <211> 56
   <212> DNA
   <213> artificial sequence
<220>
   <223> primer
<400> 20
   actgcacgct cacacggacg gtgcagtaag tcctcatcac cgacccggag agggac 56
<210> 21
   <211> 100
   <212> DNA
   <213> artificial sequence
<220>
   <223> primer
<400> 21
   actgcacgct cacacggacg ttgttgttgt tgttgttgtt gttgttgttg ttgttgttgt 60
   gcgtgcagtg cggtccacca tcaccgaccc ggacagccca 100
<210> 22
   <211> 22
   <212> DNA
   <213> artificial sequence
<220>
   <223> primer
<400> 22
   gggccaggcg cttgttggtg ta 22
<210> 23
   <211> 20
   <212> DNA
   <213> artificial sequence
<220>
   <223> primer
<400> 23
   actgcacgct cacacggacg 20
<210> 24
   <211> 20
   <212> DNA
   <213> artificial sequence
<220>
   <223> sonde
<400> 24
   tcagttcggc ggtgaggaca 20
<210> 25
   <211> 162
   <212> DNA
   <213> artificial sequence
<220>
   <223> interner standard
<400> 25
   catcaccgac ccggacagcc caccagcgtg gaccgctgtc ctcaccgccg aactgaggcc     60
   aggcggtgaa gggcaatcag ctgttgcccg tctcgctggt gaaaagaaaa accaccctgg    120
   cgcccaatac gcaaaccgcc tacaccaaca agcgcctggc cc                       162